# EUROPEAN PATENT APPLICATION

(11) **EP 4 592 405 A1**
(43) Date of publication of application: **30.07.2025**
(21) Application number: 24382078.4
(22) Date of filing: 26.01.2024
(51) Int. Cl.: C12Q 1/6886, C12Q 1/689

(54) **BACTERIAL BIOMARKERS FOR COLORECTAL CANCER DIAGNOSIS**

(71) Applicant: Servizo Galego de Saude, 15781 Santiago de Compostela (ES); Fundación Profesor Novoa Santos, 15006 A Coruña (ES); Universidade da Coruña, 15071 A Coruña (ES)
(72) Inventor: CONDE PÉREZ, Kelly, 15006 A Coruña (ES); POZA DOMÍNGUEZ, Margarita, 15006 A Coruña (ES); BOU ARÉVALO, Germán, 15781 Santiago de Compostela (ES); TRIGO TASENDE, Noelia, 15071 A Coruña (ES)
(74) Representative: Hoffmann Eitle

(57) **Abstract**

The present invention relates to a bacterial biomarker or to a combination of bacterial biomarkers in intestinal or stool samples which on one hand help in the diagnosis of colorectal cancer, and on the other hand predict the progression of colorectal cancer, as well as the response to treatment of a subject suffering from this disease.

## Description

### Technical Field of the Invention

The present invention relates to a bacterial biomarker or to a combination of bacterial biomarkers in intestinal or stool samples which on one hand help in the diagnosis of colorectal cancer, and on the other hand predict the progression of colorectal cancer, as well as the response to treatment of a subject suffering from this disease.

### State of the Art

Colorectal cancer (CRC) is one of the most common types of cancer worldwide, after breast, prostate and lung cancers (considering both sexes and all ages). Nevertheless, it is the second type of cancer that causes the most deaths worldwide, with 935173 deaths in 2020 [1]. In Spain, CRC is a malignant neoplasm with the highest incidence [2]; specifically, the province of A Coruña (Galicia, NW of Spain) is an area where the incidence and mortality of CRC are increasing every year [2]. The usual precursors of CRC are colorectal polyps and the progression from benign polyps to carcinomas, which occurs very slowly and sporadically, usually taking approximately 10 to 15 years if there is no specific genetic syndrome (which occurs only in -5% of cases) [3]. Most common symptoms of CRC are unspecific (e.g., presence of mucus or blood in stool, abdominal or pelvic pain), and most of the time patients do not show any signs until illness worsens, so colorectal tumors are frequently diagnosed at advanced stages [4-6]. Later CRC detection decreases the survival rate and increases the morbidity of patients, with a 5-year survival rate of 75-90% if cancer is detected in early stages (e.g., stages I-II) and only -15% when CRC is diagnosed in more serious and advanced stages (e.g., stage IV) [6].

Stool tests are easy and inexpensive methods for selecting CRC high-risk individuals. In Spain, with the aim of increasing the detection of cases, CRC follow-up programs have been implemented for individuals aged 50 years and above. These programs utilize fecal occult blood tests (FOBT), which promote a downward trend in CRC mortality [7]. Nevertheless, worldwide epidemiological studies have reported that the number of individuals who debuted before 50 years old has increased [8-10] and moreover, the specificity of FOBT is quite low, being significantly higher only for advanced CRC phases (stages III-IV) [11]. In addition, if FOBT results are positive, colonoscopy is recommended, which is unnecessary in most cases where other disorders may cause rectal bleeding, involving a significant workload in hospitals. Therefore, there is an urgent need for new, more specific, and non-invasive biomarkers to enhance early detection of colorectal neoplasia.

### Brief Description of the Drawings:

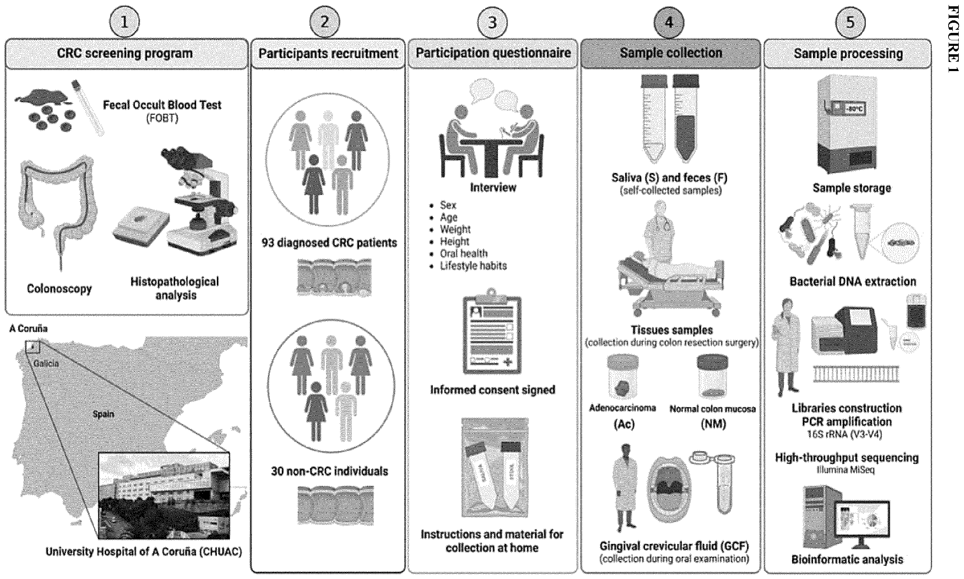

**Figure 1****.** Schematic representation of the workflow during the present study. All colorectal cancer (CRC) patients involved in this study were diagnosed and treated at the University Hospital of A Coruña (CHUAC, Spain). CRC diagnosis was based on: (1) positive colonoscopy for colorectal neoplasia confirmed by histopathological analysis of biopsied tissues or (2) through CRC screening programs consisting of a positive fecal occult blood test (FOBT) followed by a positive colonoscopy validated by histopathological analysis. The cohort for the study consisted of 93 patients diagnosed with CRC and 30 healthy individuals without any digestive disorders (non-CRC). All the participants completed a questionnaire and collected saliva (S) and fecal (F) samples at home. Tissue samples such as normal colorectal mucosa tissue (NM) and adenocarcinoma tissue (Ac) of CRC patients were collected by the surgery team after colon laparoscopic resection at CHUAC. Besides, gingival crevicular fluid samples (GCF) were collected at Pardiñas Medical Dental Clinic (A Coruña, Spain) during an oral examination. Finally, all different-nature samples were sent to the microbiology laboratory at CHUAC where they were processed, sequenced and analyzed using different bioinformatic tools.

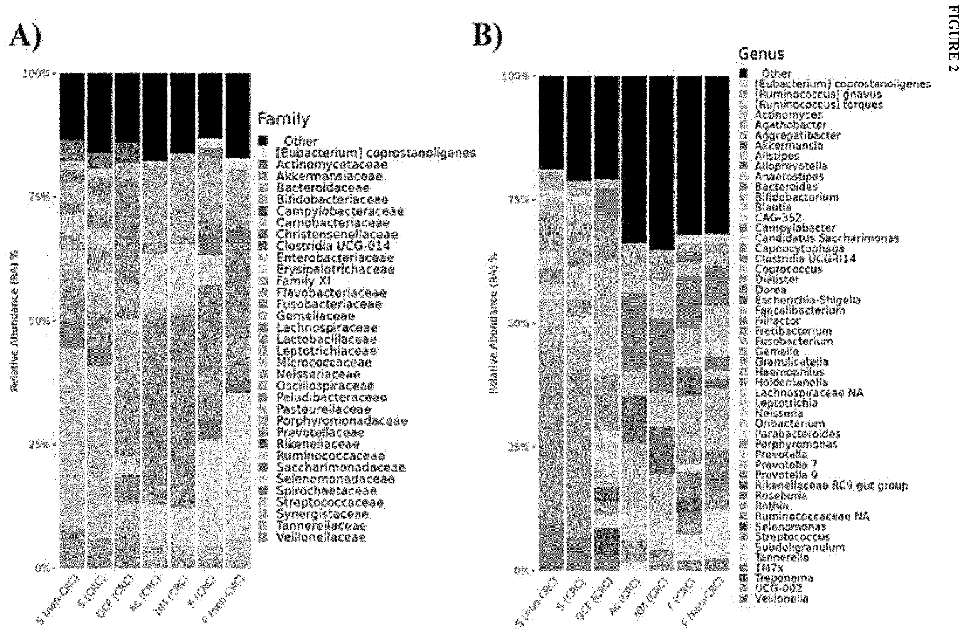

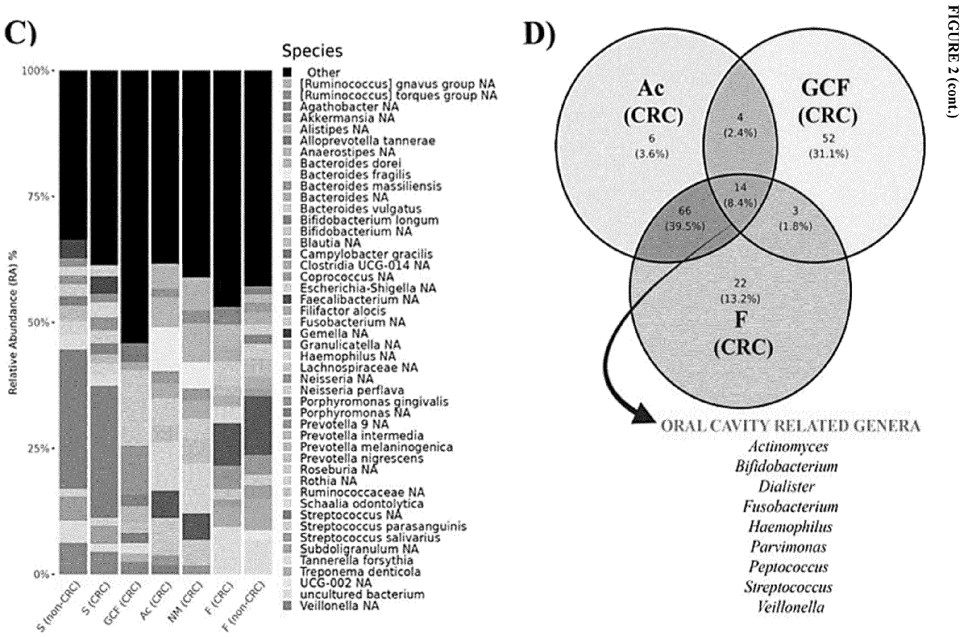

**Figures 2****.** Bacteriome landscape of the different-nature samples obtained from colorectal cancer patients (CRC) and individuals without any digestive disorders (non-CRC) analyzed by 16S rRNA Illumina sequencing. Barplots show the relative abundance (RA) at the family level (A), genera level (B) and species level (C), with a prevalence filter of 30%. The panel D shows a Venn diagram indicating the number of bacterial genera detected in each of the samples, as well as the number of genera common to all the samples with a list of oral bacteria. Samples analyzed: saliva (S) and feces (F) from both non-CRC and CRC individuals; gingival crevicular fluid (GCF), adenocarcinomas (Ac), and normal colorectal mucosa tissues (NM) from CRC patients.

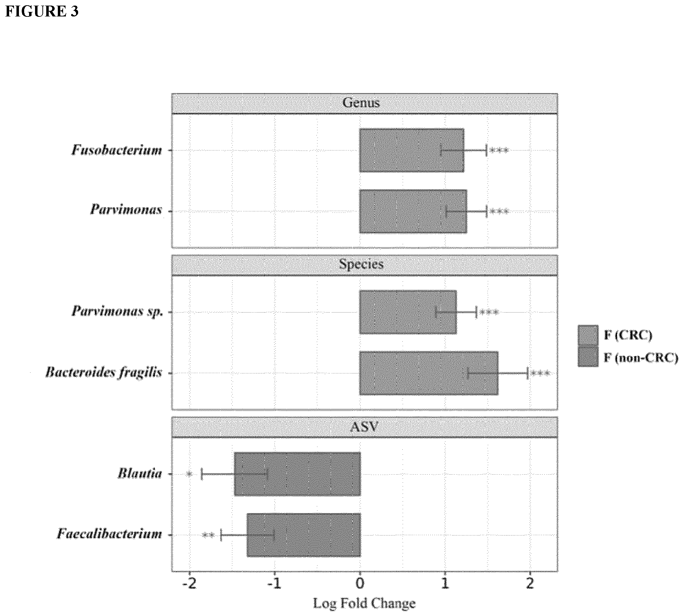

**Figure 3****.** Differential abundance analysis of the bacteriomes of fecal samples (F) from CRC and non-CRC individuals made at the genus (above), species (middle) and Amplicon Sequencing Variants (ASV, down) levels. Effect size (log fold change), standard error and adjusted p-values for each entry were obtained using the ANCOM-BC method, with a prevalence filter of 30%. Only effect sizes with adjusted p-values <0.05 are shown: (***: <0.001, **: <0.01, *: <0.05).

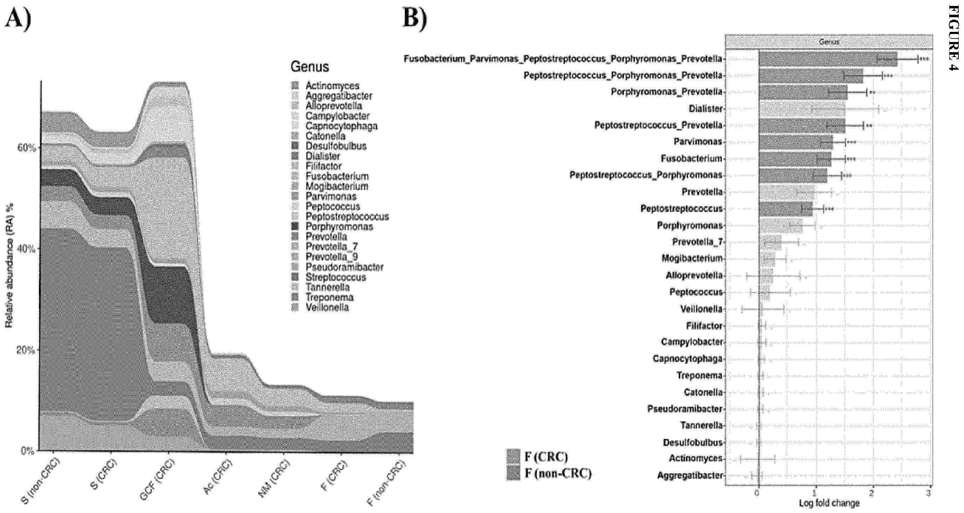

**Figure 4****.** Landscape of oral related bacteria among different-nature samples of CRC) and non-CRC individuals. A) Alluvial barplot of oral related bacterial and their relative abundance (RA) among the different samples: saliva (S) from non-CRC and CRC individuals, gingival crevicular fluid (GCF) from CRC patients, adenocarcinomas (Ac) from CRC patients, normal colorectal mucosa tissues (NM) from CRC patients and feces (F) from both non-CRC and CRC participants. B) Differential abundance analysis of groups of ASVs from typical oral related genera obtained from feces (F) from CRC and non-CRC individuals. Effect size (log fold change), standard error and adjusted p-values for each entry were obtained using the ANCOM-BC method (***: <0.001, **: <0.01, -: >0.1). No prevalence cut was used for this analysis to show unsignificant entries belonging to oral related bacteria.

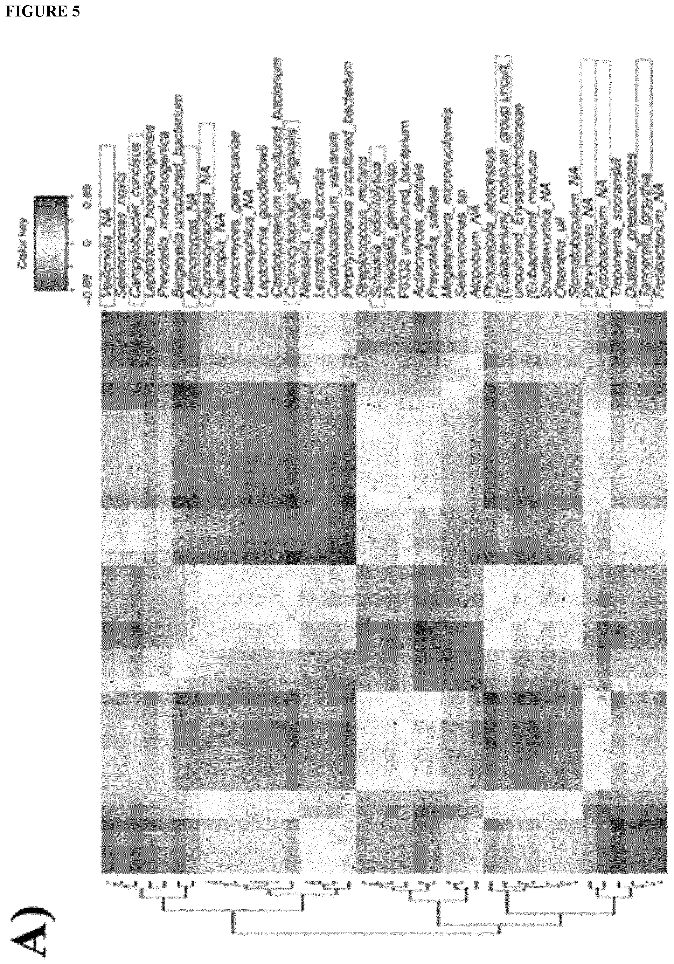

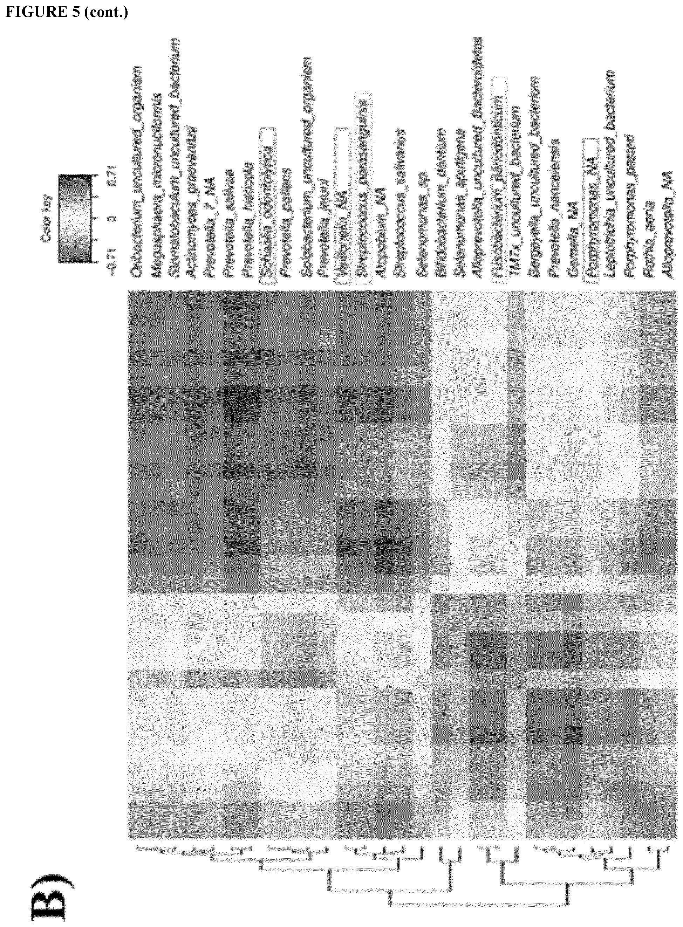

**Figure 5****.** Heat map showing the associations found among bacteria in the oral cavity of CRC patients. A) Gingival crevicular fluid samples (GCF) of CRC patients. B) Saliva samples (S) of CRC patients. Species belonging to the Socransky complexes (red, orange, green, yellow, blue and purple) are marked with rectangles of the corresponding color.

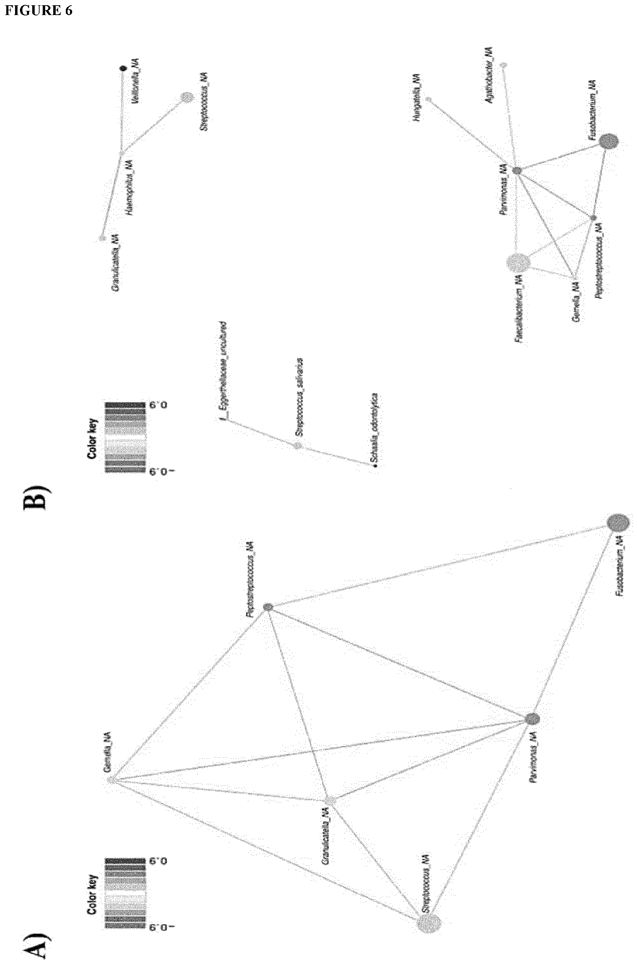

**Figure 6****.** Networks among oral bacteria present in colon tissue of CRC patients. A) Non-neoplastic colon tissue (NM). B) Colorectal adenocarcinoma tissue (Ac). Edge color corresponds to correlation strength, shown in the color key. Color of nodes corresponds to Socransky complexes color and, additionally, light blue was used for other oral related species and gray for gut associated species. Size of nodes was related to mean abundance in tissue.

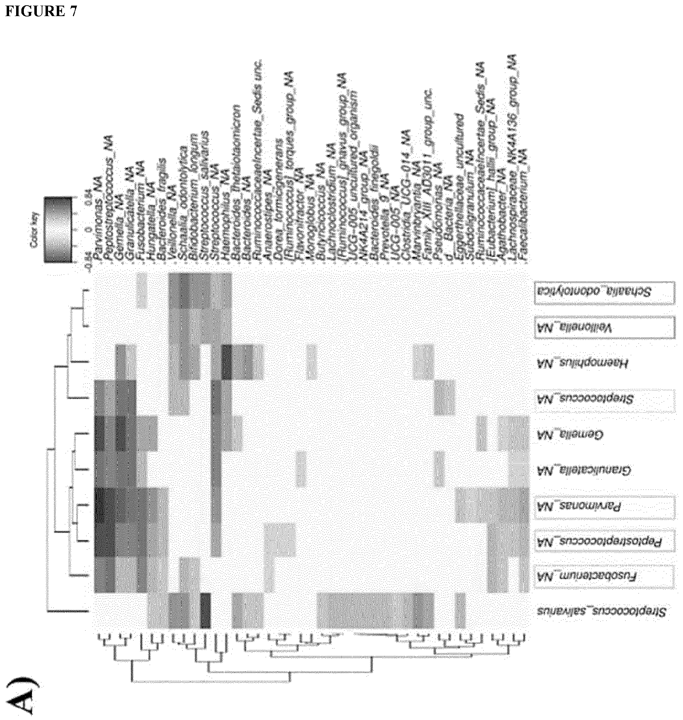

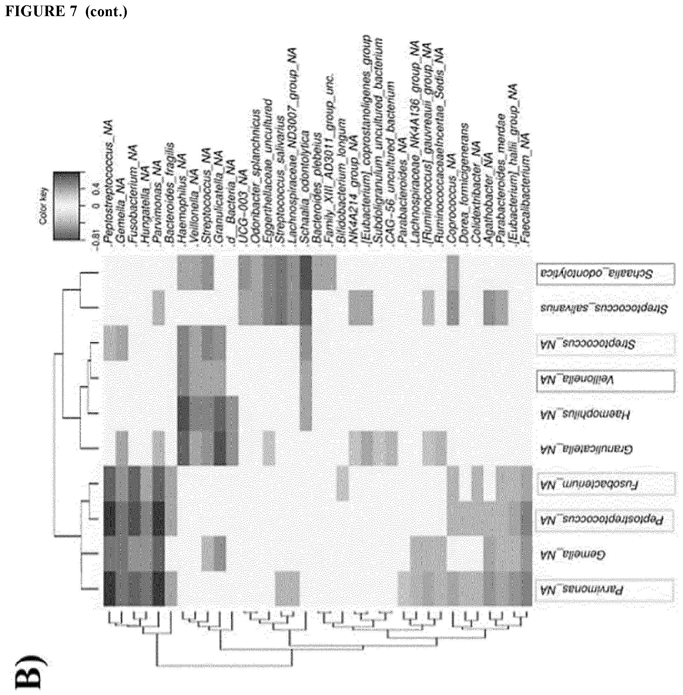

**Figure 7****.** Heat map of associations between oral-related bacteria and all the bacteria present in intestinal samples of CRC patients. A) Non-neoplastic colon tissue (NM). B) Colorectal adenocarcinoma tissue (Ac). Species belonging to the Socransky complexes were marked with rectangles of the corresponding color.

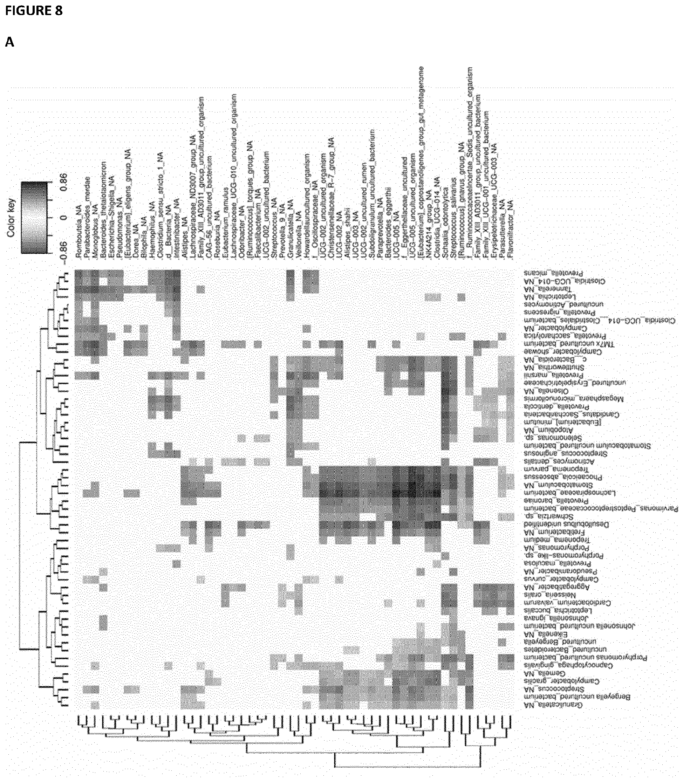

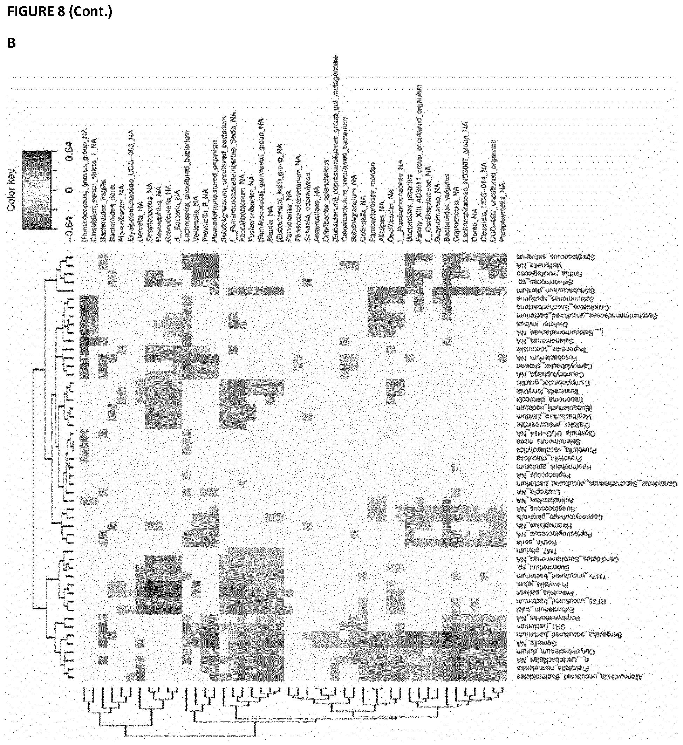

**Figure 8****.** Heat map of associations between bacteria in the oral cavity and the tumor of CRC patients. A) Gingival plaque (GCF) and adenocarcinoma (Ac) paired samples. B) Saliva (S) and Ac paired samples.

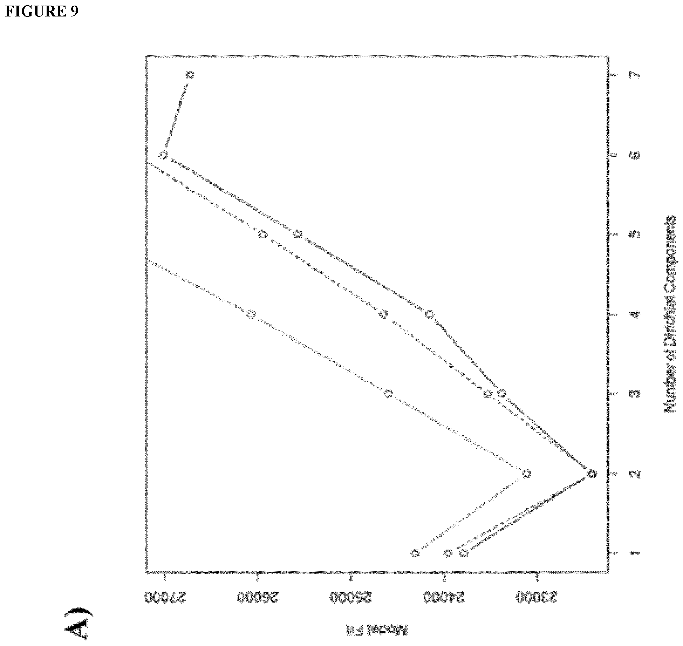

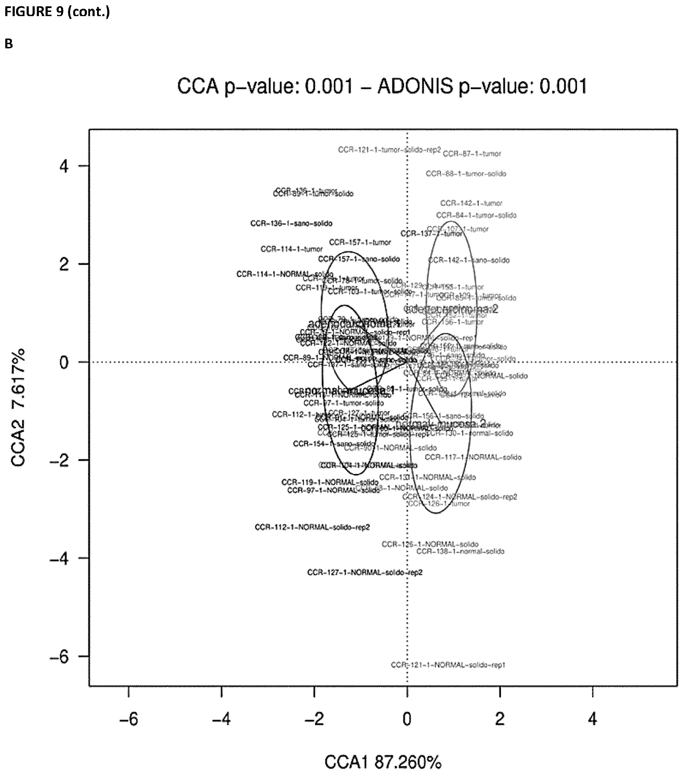

**Figure 9****.** Tissue enterotypes analysis in colorectal cancer (CRC) patients. A) Dirichlet Multinomial Mixtures (DMM) was used to infer the optimal number of community types in NM samples. Model fit was measured by Akaike's Information Criterion (aic) (dotted line), Bayesian Information Criterion (bic) (dashed line) and Laplace approximation (lplc) (solid line). B) NM samples from both enterotypes (enterotype 1: blue, enterotype 2: yellow) and their corresponding AC sample (enterotype 1: red, enterotype 2: green) were represented with a CCA plot.

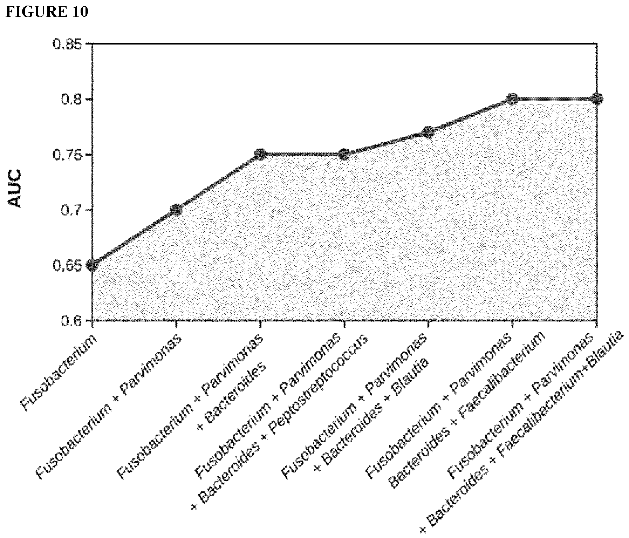

**Figure 10****.** Prediction performance of bacterial biomarkers detected in fecal samples. Prediction performance is indicated as AUC values obtained from ROC curves of a leave-one-out cross validation method based on models with 1, 2, 3 or 4 bacterial genera.

### Brief description of the invention

An aspect of the present invention refers to an *in vitro* method for determining the risk that a subject has colorectal cancer, comprising the following steps:
a. determining the levels or the concentration of bacteria belonging to the genus *Fusobacterium, Parvimonas, Bacteroides and*/*or Faecalibacterium,* or any combination thereof, in an intestinal or stool sample isolated from said subject; and
b. comparing the levels or the concentration of said bacteria in said intestinal or stool sample with one or more reference values, wherein an increase in the number of sequences of *Fusobacterium, Parvimonas, and*/*or Bacteroides,* and a decreased in the number of sequences of *Faecalibacterium,* in said sample with respect to said one or more reference values is indicative of an increase in the risk that the subject has colorectal cancer;

wherein the levels or the concentration of bacteria belonging to the genus *Fusobacterium, Parvimonas, Bacteroides and*/*or Faecalibacterium,* are determined by performing an amplification reaction from a nucleic acid preparation derived from said sample using at least one pair of primers capable of amplifying at least one representative region of said genus, and detecting the amplification product,
wherein the one or more reference values are understood to refer to the concentration and/or the total amount of each bacterium belonging to one or more of the genera proposed, in the general population or in a healthy subject.

Preferably, step a) determines the levels or the concentration of at least all of the bacteria belonging to the genus *Fusobacterium, Parvimonas, Bacteroides and Faecalibacterium,* in an intestinal or stool sample isolated from said subject.

A further aspect of the invention refers to an *in vitro* method for diagnosing colorectal cancer in a subject in need thereof, comprising the following steps:
a. determining the levels or the concentration of bacteria belonging to the genus *Fusobacterium, Parvimonas, Bacteroides and*/*or Faecalibacterium,* or any combination thereof, in an intestinal or stool sample isolated from said subject; and
b. comparing the levels or the concentration of said bacteria in said intestinal or stool sample with one or more reference values, wherein an increase in the number of sequences of *Fusobacterium, Parvimonas, and*/*or Bacteroides,* and a decreased in the number of sequences of *Faecalibacterium,* in said sample with respect to said one or more reference values is indicative that the subject has colorectal cancer;

wherein the levels or the concentration of bacteria belonging to the genus *Fusobacterium, Parvimonas, Bacteroides and*/*or Faecalibacterium,* are determined by performing an amplification reaction from a nucleic acid preparation derived from said sample using at least one pair of primers capable of amplifying at least one representative region of said genus, and detecting the amplification product,
wherein the one or more reference values are understood to refer to the concentration and/or the total amount of each bacterium belonging to one or more of the genera proposed, in the general population or in a healthy subject.

Preferably, step a) determines the levels or the concentration of at least all of the bacteria belonging to the genus *Fusobacterium, Parvimonas, Bacteroides and Faecalibacterium,* in an intestinal or stool sample isolated from said subject.

A further aspect of the invention refers to an in vitro method for determining the risk that a subject has colorectal cancer, comprising the following steps:
a. determining the levels or the concentration of bacteria belonging to the genus *Fusobacterium, Parvimonas, Bacteroides and*/*or Faecalibacterium,* or any combination thereof, in an intestinal or stool sample isolated from said subject; and
b. identifying the subject as a subject at risk of developing colorectal cancer by a predictive model which correlates at least one or more of the levels identified in step (a) with representative levels of the same from samples obtained or isolated from subjects previously identified as suffering from colorectal cancer, said predictive model having been generated by training a computer with a plurality of the concentration levels of the bacteria belonging to the genus identified in step (a) from previously identified subjects having colorectal cancer, by machine learning on said plurality of levels so as to obtain representative score profiles associated with colorectal cancer.

Preferably, step a) determines the levels or the concentration of at least all of the bacteria belonging to the genus *Fusobacterium, Parvimonas, Bacteroides and Faecalibacterium,* in an intestinal or stool sample isolated from said subject.

A further aspect of the invention refers to an in vitro method for diagnosing colorectal cancer in a subject in need thereof, comprising the following steps:
a. determining the levels or the concentration of bacteria belonging to the genus *Fusobacterium, Parvimonas, Bacteroides and*/*or Faecalibacterium,* or any combination thereof, in an intestinal or stool sample isolated from said subject; and
b. identifying the subject as a subject having colorectal cancer by a predictive model which correlates at least one or more of the levels identified in step (a) with representative levels of the same from samples obtained or isolated from subjects previously identified as suffering from colorectal cancer, said predictive model having been generated by training a computer with a plurality of the concentration levels of the bacteria belonging to the genus identified in step (a) from previously identified subjects having colorectal cancer, by machine learning on said plurality of levels so as to obtain representative score profiles associated with colorectal cancer.

Preferably, step a) determines the levels or the concentration of at least all of the bacteria belonging to the genus *Fusobacterium, Parvimonas, Bacteroides and Faecalibacterium,* in an intestinal or stool sample isolated from said subject.

In a further preferred embodiment of any of the previous aspects of the invention, the amplification reaction is carried out by means of a real-time polymerase chain reaction. In particular, the levels or the concentration of bacteria belonging to the genus *Fusobacterium, Parvimonas, Bacteroides and*/*or Faecalibacterium* as identified in step (a), are determined by performing an amplification reaction from a nucleic acid preparation derived from said sample using at least one pair of primers capable of amplifying at least one representative region of said genus and detecting the amplification product.

In a further preferred embodiment of any of the previous aspects of the invention, the said method further comprises storing the results of the method in a data carrier, preferably wherein said data carrier is a computer readable medium.

A still further aspect of the present invention refers to a computer-implemented method for determining the risk that a subject has colorectal cancer, wherein said method comprises at least the comparative step (step (b)) and optionally the provision of a result as a consequence of said comparison, as these steps are defined in the method according to any of the aspects of the present invention.

A final aspect refers to a kit capable of implementing the methodology described herein.

### Detailed Description of the Invention:

In the present invention, we performed an in-depth analysis of the oral and intestinal microbiome of samples obtained from a cohort of CRC patients (gingival crevicular fluid, saliva, non-neoplastic tissues, adenocarcinoma tissues, and feces), to establish correlations between bacteria within and between niches and to provide new insights about the oral-gut connection. Correlation analyses were performed to identify the bacterial consortia present in the oral and gut environments, including the tumor tissues. The comparison between microbiomes from CRC patients and those of healthy people allowed us to describe a specific combination of bacteria that could serve as a powerful noninvasive biomarker for CRC diagnosis.

Current CRC follow-up screenings programs in Spain are based on FOBT tests, and if the result is positive, a colonoscopy is recommended [7]. Despite this, in many cases, the CRC symptoms are non-specific, and the disease is only apparent when there is rectal bleeding or acute abdominal pain, which often corresponds to an advanced tumor stage, with a higher likelihood of distant metastasis [8, 69, 5]. At this critical phase, an individual's response to chemotherapy agents and their survival could also be compromised. Therefore, there is an urgent need for new, specific, and minimally invasive biomarkers to enhance the early detection of colorectal cancer.

Over the last few years, multiple studies have demonstrated that the gut microbiome of patients with CRC is significantly unbalanced. Colorectal dysbiosis is characterized by the loss of mutualistic species, significant reduction in microbial biodiversity, and strong decline in healthy microbiota functions [30, 70, 36, 31, 25, 50]. In the current study, we identified a clear over-representation of common well-known periodontal pathogens in all intestinal samples obtained from CRC diagnosed individuals. Interestingly, this finding suggests that some of these pathobionts could potentially serve as non-invasive fecal biomarkers for CRC diagnosis (specifically, Fusobacterium and Parvimonas). Most of the oral microorganisms detected in the samples were strict anaerobes (e.g., Fusobacterium, Parvimonas, Prevotella, Peptostreptococcus and Porphyromonas), which are probably translocated from the subgingival cavity to the gut. These oral bacteria, especially Fusobacterium and Parvimonas, were also over-represented in samples from other CRC studied cohorts. Therefore, we suggest that the co-occurrence of these oral microorganisms in the gut, which are typical drivers of chronic oral inflammatory diseases, may play an important role in the development of colorectal tumors.

In this invention, we found that the abundance of Fusobacterium increased from the normal mucosa to tumors. In addition, fecal samples from CRC patients showed to be significantly more enriched in Fusobacterium when compared to feces of non-CRC individuals. Furthermore, according to our results, another oral pathogen that overgrows in the colon of patients with CRC, is Parvimonas. Furthermore, Peptostreptococcus was another enriched genus in our CRC patient cohort. Moreover, this invention further shows that a consistent depletion of Faecalibacterium and Blautia genera was observed in CRC individuals when compared to the non-CRC group. The present invention supports that there is no clear overlap in microbiome composition between oral and fecal samples obtained from non-CRC individuals. In Spain, a significant proportion of the adult population between the ages of 50 and 80 years, estimated at approximately 35%, is affected by PD [104]. In addition, recent studies revealed that patients diagnosed with PD have an increased risk of CRC development by -44% when compared to individuals with good oral health, suggesting a positive correlation between oral disorders and CRC [37, 53]. Micro-communities of various oral pathobionts, naturally found in saliva or gingival fluids [105], have the potential to migrate from the oral cavity to the gut [106, 107]. This migration can contribute to the colonization of new colorectal microenvironments, including abnormal gut structures, especially during the progression of the PD, when these pathobionts experience over-growth [37, 53]. The similarities between the colorectal epithelium and the subgingival cavity, such as similar pH and low percentage of oxygen levels, create favorable conditions for the adaptation and overgrowth of these harmful periodontal organisms [108, 109]. In particular, the increased mucosal tissue mass, as occurs in dysplasia, provides an advantage for the growth of oral microbes due to the increased supply of nutrients. Usually, the formation of multi-species consortia enhances the viability of individual bacteria, facilitates their adhesion and invasion of host cells, disrupts cell adhesive contacts, and further increases collective virulence as well as host vascular permeability [110, 111]. This could explain why inflammatory gum infections were triggered mostly by various microbes, suggesting that the microbial cluster detected in our invention, could promote a strong pro-inflammatory effect in the gut, in a similar way that occurs in the oral cavity, altering eukaryotic cell signaling pathways [76, 77, 90, 91, 82]. However, it remains unknown how CRC-associated bacteria interact in this dysbiotic environment. In this invention we observed that oral associated bacteria in tumors correlated with each other in a similar way as they did in the oral niche. Different bacterial complexes were established in the subgingival cavity by Socransky in the year 1998 [112]. Bacteria from orange and red complexes (proteolytic and strict anaerobes) were associated with PD whereas yellow and purple ones (facultative anaerobes and saccharolytic) were established as earlier colonizers and health associated. Nevertheless, it was shown that in supragingival liquid, members of both types of bacteria coexist in the biofilms [113] and bacteria from the "early colonizers" group (e.g., Streptococcus) protect strict anaerobes (e.g., Fusobacterium) from oxidative stress [114]. This synergism could explain the positive correlation among "early colonizers" and aerobes with strict anaerobes in the saliva fluid and normal colorectal tissues, observed in the current invention. It is worth mentioning that although Fusobacterium and Parvimonas correlated positively in gingival and tissue samples, they did not correlate in saliva, suggesting that the synergy between these two pathogens is not as favorable or required in this oral environment. The lower correlation between strict and facultative anaerobic bacteria in carcinomas could indicate reduced oxygen irrigation in this microenvironment. In relation to this, Galeano et al. [109] conducted a study on the spatial distribution of intratumoral bacteria in CRC tumors. Their findings demonstrated that bacterial communities tend to populate microniches that are less vascularized and, therefore, have lower oxygen pressure. Although we were able to observe that the co-occurrence patterns of oral bacteria in carcinomas were like those in the oral cavity, it is interesting to highlight that not all PD related pathogens can reach the gut environment. For example, although Fusobacterium, Parvimonas and Peptostreptoccocus are present in adenocarcinomas, Tannerella and Treponema are absent in most patients. Furthermore, when we correlated the presence of bacteria in the saliva or subgingival fluid with their presence in colorectal tumors, no clear correlations were found. Therefore, our data indicate that the cluster of oral pathobionts detected in carcinomas represents a subset of the more complex ones present in the oral cavity.

Based on our bacteriome co-occurrence data, we consider that a preferred approach for diagnosing CRC in fecal samples should not focus solely on the detection of one bacteria, but on a consortium of oral and intestinal pathogens. Therefore, we investigated the use of oral over-abundant genera in the gut of patients with CRC as potential biomarkers in fecal samples. Initially, we tested Fusobacterium as a single diagnostic biomarker, obtaining a modest prediction performance (AUC 0.65). Specifically, in our invention, we expanded this bacteriome biomarker model by integrating other over-abundant bacteria in fecal samples from CRC patients (Parvimonas and Bacteroides) and another enriched genus in non-CRC fecal samples (Faecalibacterium), resulting in an improved performance, up to an AUC of 0.8. The genera included in our model were: Fusobacterium, Parvimonas, Bacteroides and Faecalibacterium. Our analysis revealed that the combination of bacteria, including intestinal related genera (Bacteroides and Faecalibacterium), improves the discriminatory power between non-CRC and CRC individuals. Moreover, it is important to remark that, most of these bacterial genera (Fusobacterium, Parvimonas and Bacteroides) were also found to be over-represented in adenomatous polyps, which are recognized as typical CRC precursor lesions [39, 42, 32].

The data obtained in our invention thus revealed that a specific consortium of oral and gut bacteria (Fusobacterium, Parvimonas, Bacteroides and Faecalibacterium) could be used as a predictive model for the detection of malignant neoplasms in the colon, even at early stages that are not detected by colonoscopy.

It must be noted that in the context of the present invention, the genus ***Fusobacterium*** is found at higher levels in biological intestinal or stool samples in patients with CRC compared to the general population. Furthermore, it must be noted that the following sequences NR_028933.1, NR_027588.1, NR_117734.1, NR_042365.1, NR_113384.1, NR_074412.1, NR_026084.1, NR_026085.1, NR_044689.2 and NR_044820.1 (NCBI reference sequence ID) are sequences representative of this genus and are therefore useful for the identification of bacteria belonging to this genus in said samples through a PCR method or by means of any sequencing technique.

One skilled in the art will have knowledge of other sequences representative of this genus which will be used for identifying those bacteria belonging to this genus in a stool or intestinal sample. Moreover, it must be noted that in the context of the present invention the genus ***Parvimonas*** is found at higher levels in biological intestinal or stool samples in patients with CRC compared to the general population. Furthermore, it must be noted that the following sequences NR_036934.1, NR_114675.1 and NR_114338.1 (NCBI reference sequence ID) are sequences representative of this genus and are therefore useful for the identification of bacteria belonging to this genus in said samples through a PCR method or by means of any sequencing technique.

Furthermore, it must be noted that in the context of the present invention, the genus ***Bacteroides*** is found at higher levels in biological intestinal or stool samples in patients with CRC with respect to the general population. Furthermore, it must be noted that the following sequences NR_074784.2, NR_119164.1, NR_112936.1 and NR_112141.1 (NCBI reference sequence ID) are sequences representative of this genus and are therefore useful for the identification of bacteria belonging to this genus in said samples through a PCR method or by means of any sequencing technique.

One skilled in the art will have knowledge of other sequences representative of this genus which will be used for identifying those bacteria belonging to this genus in a stool or intestinal sample. Finally, it must be noted that in the context of the present invention, the genus ***Faecalibacterium*** is found at lower levels in biological intestinal or stool samples in patients with CRC with respect to the general population. Furthermore, it must be noted that the following sequences NR_028961.1, NR 178280.1, NR 178311.1, NR_178971.1, NR_178972.1 and NR_179388.1 (NCBI reference sequence ID) are sequences representative of this genus and are therefore useful for the identification of bacteria belonging to this genus in said samples through a PCR method or by means of any sequencing technique.

One skilled in the art will have knowledge of other sequences representative of this genus which will be used for identifying those bacteria belonging to this genus in a stool or intestinal sample. Therefore, a first aspect of the present invention relates to the *in vitro* use of the level or the concentration in an intestinal or stool sample of bacteria belonging to the genus Fusobacterium, Parvimonas, Bacteroides and/or Faecalibacterium, or any combination thereof, for the diagnosis/of CRC or colorectal polyps (as an early sign or marker of CRC) in a patient, or for obtaining useful data which allows said diagnosis.

An alternative embodiment of the first aspect of the invention relates to a method for the *in vitro* diagnosis or for obtaining useful data which helps in said diagnosis of a subject suspected of possibly suffering from CRC or colorectal polyps (as an early sign or marker of CRC), which method comprises using, as an indicator in an intestinal or stool sample obtained from said subject, the level or the concentration in said sample of bacteria belonging to the genus Fusobacterium, Parvimonas, Bacteroides and/or Faecalibacterium, or any combination thereof, wherein if the level or the concentration in said intestinal or stool sample of bacteria belonging to Fusobacterium, Parvimonas, Bacteroides and/or Faecalibacterium, or any combination thereof differs or varies from that present in an intestinal or stool sample obtained from a healthy subject or with respect to a reference value, it is indicative of said subject having CRC or colorectal polyps.

In a preferred embodiment of the first aspect of the invention, the method comprises using, as an indicator in an intestinal or stool sample obtained from said subject, the level or the concentration in said sample of bacteria belonging to the genus Fusobacterium, wherein if the level or the concentration in said intestinal or stool sample of bacteria belonging to said genus is increased with respect to that present in an intestinal or stool sample obtained from a healthy subject or with respect to a reference value, it is indicative of said subject having CRC or colorectal polyps.

In another preferred embodiment of the first aspect of the invention, the method comprises using, as an indicator in an intestinal or stool sample obtained from said subject, of the level or the concentration in said sample of bacteria belonging to the genus Parvimonas, wherein if the level or the concentration in said intestinal or stool sample of bacteria belonging to said genus is increased with respect to that present in an intestinal or stool sample obtained from a healthy subject or with respect to a reference value, it is indicative of said subject having CRC or colorectal polyps.

In another preferred embodiment of the first aspect of the invention, the method comprises using, as an indicator in an intestinal or stool sample obtained from said subject, the level or the concentration in said sample of bacteria belonging to the genus Bacteroides, wherein if the level or the concentration in said intestinal or stool sample of bacteria belonging to said genus is increased with respect to that present in an intestinal or stool sample obtained from a healthy subject or with respect to a reference value, it is indicative of said subject having CRC or colorectal polyps.

In another preferred embodiment of the first aspect of the invention, the method comprises using, as an indicator in an intestinal or stool sample obtained from said subject, the level or the concentration in said sample of bacteria belonging to the genus Faecalibacterium, wherein if the level or the concentration in said intestinal or stool sample of bacteria belonging to said genus is decreased with respect to that present in an intestinal or stool sample obtained from a healthy subject or with respect to a reference value, it is indicative of said subject having CRC or colorectal polyps.

In another preferred embodiment of the first aspect of the invention, the method comprises using, as an indicator in an intestinal or stool sample obtained from said subject, the level or the concentration in said sample of bacteria belonging to the genus Fusobacterium and Parvimonas, wherein if the level or the concentration in said intestinal or stool sample of bacteria belonging to said genus is increased with respect to that present in an intestinal or stool sample obtained from a healthy subject or with respect to one or more reference values, it is indicative of said subject having CRC or colorectal polyps.

In yet another preferred embodiment of the first aspect of the invention, the method comprises using, as an indicator in an intestinal or stool sample obtained from said subject, the level or the concentration in said sample of bacteria belonging to the genera Fusobacterium, Parvimonas, Bacteroides and/or Faecalibacterium, wherein if the level or the concentration in said intestinal or stool sample of bacteria belonging to the genera Faecalibacterium is decreased with respect to that present in an intestinal or stool sample obtained from a healthy subject or with respect to one or more reference values, and wherein if the level or the concentration in said intestinal or stool sample of bacteria belonging to the genera Fusobacterium, Parvimonas, and/or Bacteroides is increased with respect to that present in an intestinal or stool sample obtained from a healthy subject or with respect to a reference value, it is indicative of said subject having CRC or colorectal polyps.

In the context of the present invention, CRC refers to the abnormal and uncontrolled cell growth of the cells that form the large intestine, leading to a alteration in its original structure. Cancer cells distinguish themselves from the adjacent normal cells by their morphology and the acquisition of new cellular characteristics.In the context of the present invention, colorectal polyps are abnormal, non-malignant formations that originate in the mucosal layer of the large intestine. It is estimated that 95% of colorectal carcinomas develop from adenomatous polyps.

It must be noted that the present invention sufficiently describes the following sequences (NCBI reference sequence ID): NR_028933.1, NR_027588.1, NR_117734.1, NR_042365.1, NR_113384.1, NR_074412.1, NR_026084.1, NR_026085.1, NR_044689.2, NR_044820.1, NR_036934.1, NR_114675.1, NR_114338.1, NR_074784.2, NR_119164.1, NR_112936.1, NR_112141.1, NR_028961.1, NR_178280.1, NR_178311.1, NR 178971.1, NR_178972.1 and NR_179388.1, which allow identifying the presence as well as the concentration of each of the genera herein mentioned.

A second aspect of the present invention relates to in vitro method for determining the risk that a subject has colorectal cancer or colorectal polyps, comprising the following steps:
a. determining the levels or the concentration of bacteria belonging to the genus *Fusobacterium, Parvimonas, Bacteroides and*/*or Faecalibacterium,* or any combination thereof, in an intestinal or stool sample isolated from said subject; and
b. identifying the subject as a subject at risk of developing colorectal cancer by a predictive model which correlates at least one or more of the levels identified in step (a) with representative levels of the same from samples obtained or isolated from subjects previously identified as suffering from colorectal cancer or colorectal polyps, said predictive model having been generated by training a computer with a plurality of the concentration levels of the bacteria belonging to the genus identified in step (a) from previously identified subjects having colorectal cancer or colorectal polyps, by machine learning on said plurality of levels so as to obtain representative score profiles associated with colorectal cancer.

A preferred embodiment of the present invention refers to the in vitro method of the second aspect of the invention, wherein step a) comprises the determination of the levels or the concentration of the bacteria belonging to the following genus: *Fusobacterium, and Parvimonas,* in an intestinal or stool sample isolated from said subject.

Another preferred embodiment of the present invention refers to the in vitro method of the second aspect of the invention, wherein step a) comprises the determination of the levels or the concentration of the bacteria belonging to the following genus: *Fusobacterium, Parvimonas,* and *Bacteroides,* in an intestinal or stool sample isolated from said subject.

Another preferred embodiment of the present invention refers to the in vitro method of the second aspect of the invention, wherein step a) comprises the determination of the levels or the concentration of all of the bacteria belonging to the following genus: *Fusobacterium, Parvimonas, Bacteroides and Faecalibacterium,* in an intestinal or stool sample isolated from said subject.

A third aspect of the invention refers to an in vitro method for diagnosing colorectal cancer in a subject in need thereof, comprising the following steps:
a. determining the levels or the concentration of bacteria belonging to the genus *Fusobacterium, Parvimonas, Bacteroides and*/*or Faecalibacterium,* or any combination thereof, in an intestinal or stool sample isolated from said subject; and
b. identifying the subject as a subject having colorectal cancer by a predictive model which correlates at least one or more of the levels identified in step (a) with representative levels of the same from samples obtained or isolated from subjects previously identified as suffering from colorectal cancer, said predictive model having been generated by training a computer with a plurality of the concentration levels of the bacteria belonging to the genus identified in step (a) from previously identified subjects having colorectal cancer, by machine learning on said plurality of levels so as to obtain representative score profiles associated with colorectal cancer.

A preferred embodiment of the present invention refers to the in vitro method of the third aspect of the invention, wherein step a) comprises the determination of the levels or the concentration of the bacteria belonging to the following genus: *Fusobacterium, and Parvimonas,* in an intestinal or stool sample isolated from said subject.

Another preferred embodiment of the present invention refers to the in vitro method of the third aspect of the invention, wherein step a) comprises the determination of the levels or the concentration of the bacteria belonging to the following genus: *Fusobacterium, Parvimonas,* and *Bacteroides,* in an intestinal or stool sample isolated from said subject.

Another preferred embodiment of the present invention refers to the in vitro method of the third aspect of the invention, wherein step a) comprises the determination of the levels or the concentration of all of the bacteria belonging to the following genus: *Fusobacterium, Parvimonas, Bacteroides and Faecalibacterium,* in an intestinal or stool sample isolated from said subject.

In a preferred embodiment of any of the previous aspects of the invention, the amplification reaction is carried out by means of a real-time polymerase chain reaction.

In the context of the present invention, reference value is preferably understood to be the result of the data of a mathematical algorithm which uses the concentration and the total amount of each bacterium belonging to one or more of the genera proposed in the present invention in the general population or in a healthy subject. The best sensitivity and specificity value will automatically be proposed using the algorithm. This algorithm will provide values along with the proposed value, with the changing sensitivity and specificity of the values in order to provide researchers with more information and to allow them to decide on the best test and cut-off value for each patient or specific situation.

In the context of the present invention, the bacteria belonging to any of the genera proposed in an intestinal or stool sample isolated from said subject can be determined, in a non-limiting manner, by means of massive sequencing of the genome of the stool such that the total number of sequences of that bacterium existing in the stool is obtained together with the total number of other bacteria present in that stool sample. Preferably, said determination is performed by means of Next Generation Sequencing methods.

It will be appreciated that the term "machine learning" generally refers to algorithms that give a computer the ability to learn without being explicitly programmed, including algorithms that learn from and make predictions about data. Machine learning algorithms employed by the embodiments disclosed herein may include, but are not limited to, random forest ("RF"), least absolute shrinkage and selection operator ("LASSO") logistic regression, regularized logistic regression, XGBoost, decision tree learning, artificial neural networks ("ANN"), deep neural networks ("DNN"), support vector machines, rule-based machine learning, and/or others.

For clarity, algorithms such as linear regression or logistic regression can be used as part of a machine learning process. However, it will be understood that using linear regression or another algorithm as part of a machine learning process is distinct from performing a statistical analysis such as regression with a spreadsheet program. Whereas statistical modeling relies on finding relationships between variables (e.g., mathematical equations) to predict an outcome, a machine learning process may continually update model parameters and adjust a classifier as new data becomes available, without relying on explicit or rules-based programming.

In a particular embodiment, the second step of the third aspect of the invention is performed by a classification method, which results in identifying the subject as a subject at risk of suffering from colorectal polyps or adenomas and/or colorectal cancer.

In one embodiment, step (b) is carried out by a classification method; preferably selected from logistic regression, random forest, gradient boosting (GB), adaptive boosting (AB), extreme Gradient Boosting (XGB) k-nearest neighbors (kNN), artificial neural network (ANN), support vector machine (SVM), and combinations thereof.

In an embodiment, the predictive model is generated by training the computer with a plurality of levels or concentrations, as indicated in the second or third aspect of the invention from previously identified samples from subjects having colorectal adenomas and/or colorectal cancer by machine learning on said plurality of levels so as to obtain representative multivariable data sets associated with this group of patients; wherein the training comprises the following steps:
(i) training data, from a plurality of score profiles, is randomly stratified into:
   - a calibration dataset (for example in a percentage of about 75%), and
   - a validation dataset (for example in a percentage of about 25%);
(ii) the predictive model is seeded on the calibration dataset (particularly is developed by applying a machine learning method selected from a regression method, a classification method or a combination thereof on the calibration dataset);
(iii) the predictive model is optimized by an internal cross validation; preferably by a k-fold cross validation, wherein each of the k cases of the k-fold cross validation is used for testing only once and one at a time; and
(iv) the predictive model is further validated by predicting new samples using the validation dataset.

In some embodiments, the second step is performed by a classification method wherein the patients are assigned a probability of belonging to given category such as patients having colorectal adenomas and/or colorectal cancer. In some embodiments, the classification method is carried out by a method selected from gradient boosting, support vector machine (SVM), decision trees, K nearest neighbors, Naive Bayes or neural networks. In a preferred embodiment, the classification method is carried out by a Gradient Boosting. As used herein, Gradient Boosting is a machine learning algorithm that uses a gradient boosting framework. Gradient Boosting trees, a decision-tree-based ensemble model, differ fundamentally from conventional statistical techniques that aim to fit a single model using the entire dataset. Such ensemble approach improves performance by combining strengths of models that learn the data by recursive binary splits, such as trees, and of "boosting", an adaptive method for combining several simple (base) models. At each iteration of the gradient boosting algorithm, a subsample of the training data is selected at random (without replacement) from the entire training data set, and then a simple base learner is fitted on each subsample. The final boosted trees model is an additive tree model, constructed by sequentially fitting such base learners on different subsamples. This procedure incorporates randomization, which is known to substantially improve the predictor accuracy and also increase robustness. Additionally, boosted trees can fit complex nonlinear relationships, and automatically handle interaction effects between predictors as addition to other advantages of tree-based methods, such as handling features of different types and accommodating missing data. Hence, in many cases their predictive performance is superior to most traditional modelling methods. In a particular embodiment, the second step is performed by a regression method; preferably selected from multiple linear regression (MLR), principal component regression (PCR), partial least squares regression (PLSR), artificial neural network (ANN), support vector machine (SVM), random forest (RF), lassor regression, ridge regression and combinations thereof.

In a particular embodiment, the second step is performed by a classification method, more in particular, by gradient boosting, which includes the value of one or more variables of the gene expression profile collected in step (i) and which contribute to the identification of the patients having colorectal adenomas and/or colorectal cancer.

In a particular embodiment, the second step is performed by a regression method which includes the value of one or more variables of the score profile collected in step (i) and which contribute to the identification of the patients having colorectal adenomas and/or colorectal cancer.

In another preferred embodiment of the first, second or third aspect of the invention or of any of the preferred embodiments of the invention, said levels or concentration of bacteria refer to the total amount of the bacteria belonging to the genus with respect to the total bacteria present in said sample.

In another preferred embodiment of the first, second or third aspect of the invention or of any of the preferred embodiments of the invention, the levels or the concentration of bacteria belonging to the genera *Fusobacterium, Parvimonas, Bacteroides and*/*or Faecalibacterium,* are determined by performing an amplification reaction from a nucleic acid preparation derived from said sample using a pair of primers capable of amplifying one or more representative regions of said genera. In yet another preferred embodiment of the first, second or third aspect of the invention or of any of the preferred embodiments of the invention, the amplification reaction is carried out by means of a real-time polymerase chain reaction. Preferably, the detection of the amplification product is carried out by means of a fluorescent intercalating agent. Even more preferably, the detection of the amplification product(s) is carried out by means of a labeled probe, wherein the probe preferably comprises at its 5' end a reporter pigment and at its 3' end a "quencher" pigment or silencer or buffering agent.

In a fourth aspect of the invention, the method of the first, second or third aspect of the invention or of any of the embodiments thereof further comprises storing the results of the method in a data carrier, wherein said data carrier is preferably a computer readable medium.

In a fifth aspect of the invention, the method of the first, second or third aspect of the invention or of any of the embodiments thereof comprises at least the implementation of the comparative step and optionally the provision of a result as a consequence of said comparison using a computer program.

A sixth aspect of the invention relates to a kit comprising one or more pairs of primers capable of amplifying bacteria belonging to the genera *Fusobacterium, Parvimonas, Bacteroides and*/*or Faecalibacterium.* Preferably and as mentioned, a sixth aspect of the invention relates to a method for the detection of bacteria from a stool or intestinal sample, comprising the following steps:
i) contacting the sample to be analyzed with a reaction mixture containing specific primers capable of amplifying bacteria belonging to the genera *Fusobacterium, Parvimonas, Bacteroides and*/*or Faecalibacterium* preferably for performing multiplex PCR,
ii) performing amplification by means of polymerase chain reaction,
iii) identifying the formation of the products of the preceding step, said formation being indicative of the levels or the concentration of bacteria belonging to one or more of the genera *Fusobacterium, Parvimonas, Bacteroides and*/*or Faecalibacterium.*

In relation to this sixth aspect of the invention, it preferably provides a method for simultaneously detecting *Fusobacterium, Parvimonas, Bacteroides and*/*or Faecalibacterium.*

In a particular embodiment of this sixth aspect of the invention, DNA fragments included or comprised in sequences 1 to 12 are amplified.

In another embodiment of this sixth aspect of the invention, the amplification products which allow identifying the different bacterial species and groups are detected by means of using probes. In a more preferred embodiment, these probes have a length between 15 and 25 nucleotides. The primers can be designed by means of multiple alignment with programs such as CLUSTAL X, which allow identifying highly conserved regions that serve as a template.

Given the great abundance of PCR inhibitors, such as humic and fulvic acids, heavy metals, heparin, etc., which may give rise to false negatives, and despite the existence of methods which reduce the concentration of molecules of this type, it is advisable (cf. J. Hoorfar et al., "Making internal amplification control mandatory for diagnostic PCR" J. of Clinical Microbiology, Dec. 2003, pp. 5835) for PCR assays to contain an internal amplification control (IAC). This IAC is simply a DNA fragment which is amplified simultaneously with the target sample, such that its absence at the end of the assays is indicative of the presence of factors which have led to an unwanted PCR development.

Throughout the description, the term "specific" means that the primers comprise a nucleotide sequence that is completely complementary to the genes or gene fragments used by the present invention.

A preferred embodiment of the sixth aspect of the invention relates to the use of the kit or of the methodology therein described for implementing the methodology according to any of the first, second, or third aspects of the present invention.

The following examples merely illustrate the present invention and must not be understood as limiting same.

### Examples of the Invention:

### Example 1:

### MATERIALS AND METHODS

### Patient's recruitment

A total of 159 patients with CRC were recruited. To select CRC patients for this study, several exclusion criteria were established: (1) no antibiotic intake in less than one month, (2) no infectious disease in the last three months, (3) no chemotherapy and/or radiotherapy treatments prior to sample collection, (4) no genomic predisposition to develop CRC (other cases of CRC in first-degree relatives or Lynch syndrome among others), (5) no diagnosis of other gut disorders (such as inflammatory bowel disease), (6) no immunological diseases and (7) no transplants and/or any immunosuppressortreatment. Based on these criteria only 93 of the initial 159 patients were included. Likewise, CRC patients' companions/couples were asked to participate in the study and only 34 agreed to participate. Individuals included in this non-CRC control cohort followed the same exclusion criteria as CRC patients but were not diagnosed with any type of cancer. The rigorous selection process performed in the study ensured that the control group consisted of individuals who were free from cancer, making it a suitable comparison cohort for studying the microbiome in the context of CRC. Only 30 of the 34 individuals met the requirements. Informed consent was obtained from all the participants before the sample collection phase.

### Sample collection

A total of 93 samples of ca. 20 mL of feces (F) and 93 samples of 5 mL of unstimulated saliva (S) were collected at home by CRC patients included in the study, before starting the low-residue diet required for laparoscopy. Thirty healthy individuals were recruited from the same samples: F (n = 30) and S (n = 28). A previous interview was conducted with all participants to recover data related to age, sex, weight, height, and lifestyle habits, such as diet or sporting activity, oral diseases, antibiotic, intake, and previous surgeries. Furthermore, the F samples were kept in the presence of 10 mL of RNAlater reagent (Thermo Fisher Scientific, Waltham, Massachussets, USA). Gingival crevicular fluid (GCF) samples (n = 19) from CRC patients were collected by inserting sterile paper points (ISO 30, Henry Schein, USA) in the subgingival sulcus of different teeth for 30 s. Eight paper points per patient were obtained and placed in an Eppendorf tube containing 500 µL of RNAlater (Thermo Fisher Scientific, Waltham, Massachussets, USA). A total of 56 adenocarcinoma tissue samples from the colon (Ac) and 58 non-neoplastic colon tissue samples from the surrounding areas (NM) were collected via sterile dissection during surgical resection. A total of 2 g of amoxicillin/clavulanic acid was administered to the patients at the beginning of the laparoscopic and postoperatory stages (a total of 3 doses every 8 h). All tissue samples were immediately stored in the presence of 500 µL of RNAlater reagent (Thermo Fisher Scientific, Waltham, Massachussets, USA) for nucleic acid extraction and sequencing. All samples were stored at -80 °C until further analysis.

### Oral and dental check-up

As cited above, GCF samples from CRC patients who agreed to undergo an oral examination were collected by a periodontist at the Pardiñas Medical Dental Clinic (A Coruña, Galicia, Spain). Only 19 of 93 went to the dental check-ups. Periodontal assessment for each patient was done according to the 2017 World Workshop on the Classification of Periodontal and Peri-Implant Diseases and Conditions [54]. The gingival condition of each patient was recorded using the Silness-Loe gingival index [55]. The Decayed, Missing, and Filled Teeth (DMFT) index was assessed for each patient using intraoral examination and Cone-beam Computed Tomography (CBCT; Carestream Dental LLC, Atlanta, USA). The presence or absence of periapical lesions was ruled out by CBCT analysis in each patient.

### Bacterial DNA extraction

### Stool and saliva samples

First, F and S samples of CRC and non-CRC individuals were defrosted and then centrifuged (2 min at 5000 rpm and 4 °C). Next, 2 mL of each supernatant were centrifuged again (10 min at 13000 rpm at 4°C). Final pellets were resuspended in 100 µL of nuclease-free water. To induce cellular wall lysis of all bacteria, samples were incubated (37°C, at 400 rpm, 1 h) in presence of 5 µL of an enzymatic cocktail (EC) containing 20 mg/mL of lysozyme, 1.25 KU/mL of lysostaphin and 0.625 KU/mL of mutanolysin (all enzymes from Sigma-Aldrich, St. Louis, Missouri, USA). Bacterial DNA from F and S samples was extracted using the MasterPureTM Complete DNA and RNA Purification Kit (Epicentre, Madison, Wisconsin, USA).

### Tissue and gingival crevicular fluid samples

Bacterial DNA from tissue samples was extracted from 20 mg of colon mucosa (non-neoplastic and adenocarcinoma tissues) using the AllPrep^{®} DNA/RNA Mini kit (Qiagen, Hilden, Germany). Tissue homogenization was performed using Lysing Matrix E tubes (MP Biomedicals, St. Ana, California, USA) and a 1600 MiniG system (Thermo Fisher Scientific, Waltham, Massachussets, USA) at 1500 rpm for 10 min. A total of 30 µL of EC was added to samples after homogenization. In the GCF samples, only a 2 min vortex at high speed was used to detach bacteria from endodontic paper points. The paper points were carefully discarded, and 500 µL of sterile phosphate buffered saline was added. A new centrifugation at 13000 rpm for 30 min at 4 °C was carried out and the bacterial pellet was used to extract bacterial DNA following the AllPrep^{®} DNA/RNA Mini kit manufacturer's instructions with an additional enzymatic lysis (30 µL of EC). For all samples, the final DNA was eluted in EB buffer (Qiagen, Hilden, Germany) and stored at -20 °C until library preparation. Negative controls were used for each extraction were done to avoid contamination.

### 16S rRNA metabarcoding

For microbial identification, two hypervariable regions of the 16S rRNA gene (V3-V4) were amplified by PCR using 5 ng/µL of DNA and the following oligonucleotides: 5' TCGTCGGCAGCGTCAGATGTGTATAAGAGACAGCCTACGGGNGGCWGCAG as the forward primer and 5' GTCTCGTGGGCTCGGAGATGT GTATAAGAGACAGGACTACHVGGGTATCTAATCC as the reverse primer [56].

All libraries were prepared following the Illumina 16S Metagenomic Sequencing Library Preparation protocol (Illumina, San Diego, California, USA). The pooled final libraries were diluted to a final concentration of 10 pM, and and 20% of 10 pM PhiX (Illumina, USA) was added for sequencing using paired end Illumina MiSeq v3 reagent kits 2×300 (Illumina, San Diego, California, USA). DNA quantification was performed using the Qubit dsDNA HS Assay Kit (Invitrogen, Waltham, Massachusetts, USA), and the library size was checked using a 2100 Bioanalyzer Instrument (Agilent Technologies, St. Clara, California, USA). Negative controls were included in all cases to avoid contamination.

### Bioinformatic analysis

The quality of all FASTQ files generated from 16S rRNA gene sequencing was checked using FastQC [57]. Afterwards, sequences were analyzed using QIIME2 (version 2021.11) [58] on a per sequencing run basis, utilizing DADA2 to trim, denoise, correct sequencing errors and remove chimeras, resulting in several tables of Amplicon Sequence Variants (ASVs) [59]. The resulting features from each sequencing run were then merged and collapsed into a single feature table and classified using the SILVA 138 99% reference database [60] through QIIME2. Afterwards, R (version 4.1) [61] and Phyloseq (version 1.36.0) [62] were used to create a Phyloseq object from QIIME 2 results, to process it and to clean it. Mainly, filtering ASVs to those from the bacterial kingdom, subtracting the raw count of ASVs that appeared in control samples from the rest on a per sequencing run basis and removing ASVs in specific genera that are typically involved in reagent contamination [63]. Additionally, taxonomy levels were propagated (e.g., An ASV that was classified at the genus level but not classified at the species level had their last known level propagated, filling the species level with Genus_NA). To calculate the mean relative abundance for each type of sample the cleaned and rarified Phyloseq was used. Filters composed of a minimum abundance of 0.01% and minimum prevalence of 30% were applied, firstly in a per biosample basis (each biosample can be sequenced multiple times) and then in a per group basis (e.g., feces of CRC patients), obtaining the mean relative abundance in multiple taxonomy levels. Alpha and beta diversity analyses were also performed through R and Phyloseq. Differential abundance analysis (DAA) was performed using R package ANCOM-BC (version 2.0.1) [64] on the cleaned, non-rarified Phyloseq object, adjusting the p-values by the Holm-Bonferroni method [65] and using a prevalence cut of 30%, except in the test of agglomerations of oral genera, in which prevalence filter was removed to show every genus of interest.

To perform the co-occurrence study raw counts were normalized with ANCOM-BC [64] and species with less than 0.01% of mean abundance or in less than 30% of the samples were filtered out. Paired samples (S, GCF, NM, and Ac from the same CRC individuals) were used to study the intra- and inter-niche correlations at the bacterial level. To assess the correlations among the bacteria in the oral samples, unsupervised sPCA from mixOmics R package was performed. To elucidate the associations of oral bacteria in tumors a multivariate analysis (sPLS-canonical) from the mixOmics R package was applied using the normalized dataset of all bacterial counts from the tissue sample and a subset of the oral-associated bacteria present in the samples [66]. Normalized data from 47 MN tissues were used as input for DMM algorithm [67] to identify the optimal number of clusters (tissue enterotypes) based on Laplace approximation. Subsequently, Bray Curtis distances were calculated and for graphical visualization, an ordination technique was performed with non-metric multidimensional scaling (NMDS) method [68]. Taxonomic differences among enterotypes were assessed using Wilcoxon rank sum paired tests and canonical correlation analysis (CCA) was used to plot these differences using R [61].

Logarithmic regression models were used to evaluate the discriminatory capacity of bacteria as biomarkers in feces. Accuracy was evaluated using Receiver Operating Characteristic (ROC) curves and the area under the curve (AUC) was calculated and validated using the leave -one-out cross-validation (LOOCV) method. Additionally, the bootstrapping algorithm implemented in the Boruta R library (Kursa and Rudnicki, 2010) was used to blind the selection of additional biomarkers. The analysis was performed at the genus level.

### RESULTS

### CRC patients and healthy controls characteristics

A total of 93 patients with CRC and 30 healthy individuals (non-CRC) were included in the present study. CRC diagnosis was confirmed by colonoscopy and histopathological analysis in all cases. The characteristics of both groups are summarized in Tables 1 and 2. Figure 1 summarizes the workflow of this study.

**Table 1. Characteristics of the colorectal cancer patients' cohort (CRC, n = 93) and the control group (non-CRC; n = 30).**

| | | **Colorectal cancer** | **Control** | **Total** | ***p-*value** |
|---|---|---|---|---|---|
| | | (CRC) | (non-CRC) | (CRC + non-CRC) | |
| **Sex** | **Females** | 36 (38.71) | 23 (76.67) | 59 (47.97) | 0.020 |
| n (%) | **Males** | 57 (61.29) | 7 (23.33) | 64 (52.03) | |
| **Age** (years) | **Females** | 65 | 63 | 65 | 0.107 |
| Median | **Males** | 70 | 64 | 67 | 0.053 |
| **Height** (cm) | **Females** | 170 | 160 | 160 | 0.984 |
| Median | **Males** | 169 | 167.5 | 169 | 0.281 |
| **Weight** (kg) | **Females** | 71 | 67 | 68 | 0.491 |
| Median | **Males** | 77 | 82 | 77.5 | 0.635 |
| **Location** | **Cecum** | 6 (6.45) | - | 6 (6.45) | - |
| **n (%)** | **Ascending colon** | 29 (31.18) | - | 29 (31.18) | |
| | **Hepatic flexure** | 1 (1.07) | - | 1 (1.07) | |
| | **Transvers e colon** | 3 (3.22) | - | 3 (3.22) | |
| | **Splenic flexure** | 2 (2.15) | - | 2 (2.15) | |
| | **Descendin g colon** | 22 (23.65) | - | 22 (23.65) | |
| | **Sigmoid colon** | 18 (19.35) | - | 18 (19.35) | |
| | **Rectum** | 10 (10.75) | - | 10 (10.75) | |
| | **Undetermi ned** | 2 (2.15) | - | 2 (2.15) | |
| **CRC Stage (TNM) %** | **T** [1-4] | **T1** (17.20); **T2** (12.90); **T3** (62.36); **T4** (5.38) | - | **T1** (17.20); **T2** (12.90); **T3** (62.36); **T4** (5.38) | |
| | **N** [0-5] | **N0** (64.51); **N1** (17.20); **N2** (7.53); **N3** (2.15); **N4** (2.15); **N5** (2.15) | - | **N0** (64.51); **N1** (17.20); **N2** (7.53); **N3** (2.15); **N4** (2.15); **N5** (2.15) | - |
| | **M** [0-3] | **M0** (92.47); **M1** (2.15); **M2** (2.15); **M3** (1.07) | - | **M0** (92.47); **M1** (2.15); **M2** (2.15); **M3** (1.07) | |
| | **Undetermi ned** | 2.06 | - | 2.06 | |
| **Bristol Stool Scale** | **Type 1** | 0(0) | 0 (0) | 0(0) | 0.399 |
| **n (%)** | **Type 2** | 8 (8.60) | 3 (10) | 11 (8.94) | |
| | **Type 3** | 10 (10.76) | 1 (3.33) | 11 (8.94) | |
| | **Type 4** | 38 (40.86) | 19 (63.34) | 57 (46.35) | |
| | **Type 5** | 8 (8.60) | 3 (10) | 11 (8.94) | |
| | **Type 6** | 10 (10.75) | 1 (3.33) | 11 (8.94) | |
| | **Type 7** | 5 (5.38) | 1 (3.33) | 6 (4.88) | |
| | **Undetermi ned** | 14 (15.05) | 2 (6.67) | 16 (13.01) | |
| **Periodonta l Disease (PD)** | **No** | 33 (35.48) | 14 (46.67) | 47 (38.21) | 0.535 |
| n (%) | **Yes** | 54 (58.07) | 15 (50) | 69 (56.10) | |
| | **Undetermi ned** | 6 (6.45) | 1 (3.33) | 7 (5.69) | |
| **Physical Activity** | **No** | 44 (47.31) | 21 (70) | 65 (52.85) | 0.083 |
| n (%) | **Yes** | 40 (43.01) | 7 (23.33) | 47 (38.21) | |
| | **Undetermi ned** | 9 (9.68) | 2 (6.67) | 11 (8.94) | |
| **Alcohol Consumpti on** | **No** | 54 (58.06) | 21 (70) | 75 (60.98) | 0.560 |
| n (%) | **Yes** | 30 (32.26) | 7 (23.33) | 37 (30.08) | |
| | **Undetermi ned** | 9 (9.68) | 2 (6.67) | 11 (8.94) | |
| **Tobacco Consumpti on** | **No** | 70 (75.27) | 22 (73.33) | 92 (74.8) | 0.788 |
| n (%) | **Yes** | 14 (15.05) | 6 (20) | 20 (16.26) | |
| | **Undetermi ned** | 9 (9.68) | 2 (6.67) | 11 (8.94) | |
| **Caffeine Consumpti on** | **No** | 66 (70.97) | 19 (63.93) | 85 (69.11) | 0.511 |
| n (%) | **Yes** | 18 (19.35) | 9 (29.40) | 27 (21.95) | |
| | **Undetermi ned** | 9 (9.68) | 2 (6.67) | 11 (8.94) | |
| **Sleep Disorders** | **No** | 58 (62.37) | 23 (76.67) | 81 (65.85) | 0.280 |
| n (%) | **Yes** | 24 (25.81) | 6 (20) | 30 (24.39) | |
| | **Undetermi ned** | 11 (11.82) | 1 (3.33) | 12 (9.76) | |
| **Environm ent** | **City** | 31 (33.33) | 18 (60) | 49 (39.84) | 0.551 |
| (Most habitual) | **Countrysi de** | 45 (48.39) | 8 (26.67) | 53 (43.09) | |
| n (%) | **Both** | 8 (8.60) | 3 (10) | 11 (8.94) | |
| | **Undetermi ned** | 9 (9.68) | 1 (3.33) | 10 (8.13) | |

**Table 2. Oral health characteristics of the colorectal cancer (CRC) patients who attended the oral and dental check-up.**

| **Colorectal Cancer (CRC)** | | | | |
|---|---|---|---|---|
| | | | **n** | % |
| **Periodontal Disease** (PD) | **Gingivitis** | **IA** | 1 | 5.26 |
| | | **IB** | 1 | 5.26 |
| | **Initial Periodontitis** | **IIA** | 3 | 15.79 |
| | | **IIB** | 3 | 15.79 |
| | **Mild Periodontitis** | **IIIA** | 4 | 21.06 |
| | | **IIIB** | 1 | 5.26 |
| | **Progressive Periodontitis** | **IVA** | 0 | 0 |
| | | **IVB** | 3 | 15.79 |
| | - | **No PD** | 2 | 10.53 |
| | | **No teeth¹** | 1 | 5.26 |
| **Gingival Index²** | | **0.1 - 1** | 7 | 36.84 |
| | | **1.1 - 2** | 10 | 52.64 |
| | | **2.1 - 3** | 1 | 5.26 |
| | | **No teeth¹** | 1 | 5.26 |
| **Tooth Loss** (Number of missing teeth) | | **1-5** | 9 | 47.36 |
| | | **6-10** | 4 | 21.06 |
| | | **11-15** | 4 | 21.06 |
| | | **> 15** | 1 | 5.26 |
| | | **No teeth¹** | 1 | 5.26 |
| **Caries Index** (DMFT Index: decayed,missing, filled) | | **1-10** | 4 | 21.06 |
| | | **11-20** | 12 | 63.15 |
| | | **> 20** | 2 | 10.53 |
| | | **No teeth¹** | 1 | 5.26 |
| **Periapical lesion** | | **No** | 14 | 73.68 |
| | | **Yes** | 5 | 26.32 |

| | | | | |
|---|---|---|---|---|
| **¹No teeth:** colorectal cancer (CRC) patient with completely missing teeth due to acute periodontitis (dental implants only). **²Gingival Index: Level 0.1-1:** Mild degree of gingival inflammation, slight gingival color change, no bleeding. **Level 1.1** - **2:** Moderate degree of inflammation, gingival reddening and swelling, gingival bleeding on probing and pressure. **Level 2.1** - **3:** Strong level of inflammation, intense gingival reddening and swelling, plentiful bleeding, and the possibility of ulceration. | | | | |

A total of 377 samples were sequenced and analyzed using different bioinformatic procedures. In the CRC group, 93 fecal samples (F), 93 saliva samples (S), 19 gingival crevicular fluid samples (GCF), 58 normal colorectal mucosa samples (NM) and 56 adenocarcinoma samples (Ac) were obtained. For the non-CRC group, 30 F and 28 S samples were processed and analysed. The colon distribution of the different Ac samples studied in the CRC patients revealed that 31.18% were located in the ascending colon, 23.65% in the descending colon and 19.35% in the sigmoid colon (Table 1). Most of the CRC patients of this study were diagnosed in advanced stages, being the 62.36% of them in the T3 stage and 5.38% in the T4 stage (Table 1). Nevertheless, 64.51% of the CCR patients had no lymph node invasion (N0) and 92.47% of the patients had no distant metastasis (Table 1). Interestingly, a high percentage of patients with CRC (58.07%) were admitted having oral disorders to the questionnaire (e.g., halitosis, gingivitis, periodontitis, and tartar on teeth or caries, among others). In contrast, only 23.33% of the individuals in the non-CRC control group presented dental or gum disorders (Table 1). Specifically, among CRC patients who agreed to attend the oral check-up, 89.47% had periodontal disease in early or advanced stages (Table 2). Further characteristics of the studied cohorts (CRC and non-CRC) are summarized in Tables 1 and 2.

### Microbiome composition of CRC patients

Microbiome analysis of different samples obtained from a cohort of patients with CRC was performed (Figure 1). To achieve this, the S and F samples from CRC patients were compared with samples from non-CRC individuals. The median number of reads per sample across sequencing runs was 49737 (21 million reads in total), decreasing to a median of 32104 (13 million reads in total) after quality control and DADA2 processing.

This resulted in 15213 Amplicon Variant Sequences (ASVs) with a median length of 418 bp. Regarding the oral cavity microbiome of CRC patients, important periodontal pathobionts were identified specifically in GCF samples (Figure 2), being the most represented: Fusobacterium sp. (15%), Porphyromonas gingivalis (9.48%), Prevotella intermedia (3.20%), Prevotella nigrescens (2.10%), Tannerella forsythia (2.03%), Alloprevotella tannerae (1.72%), Treponema denticola (1.65%) and the emerging periodontal pathogen Filifactor alocis (1.58%) (Figure 2C). Regarding the gut microbiome, and focusing on F samples, Ruminococcaceae, Lachnospiraceae and Bacteroidaceae were the most abundant families in the two groups (CRC and non-CRC) as shown in Figure 2A. However, Prevotellaceae, Enterobacteriaceae and Rikenellaceae were more abundant in the CRC group than in the non-CRC group (Figure 2A). Interestingly, the Fusobacteriaceae family was significantly over-enriched in the CRC samples (adjusted p-value <0.001). In contrast, in the F samples from the healthy group, the families Oscillospiraceae, Streptococcaceae and Bifidobacteriaceae were more represented compared to F samples in CRC patients (Figure 2A). Consequently, Fusobacterium and Parvimonas genera in fecal samples of CRC patients were significantly over-abundant (adjusted p-values <0.001 in both cases) compared to F samples of the non-CRC group (Figure 3). Notably, when taking sex into account these differences were still visible, being more abundant in both males and females diagnosed with CRC. More specifically, at the species level, Parvimonas sp., an important periodontal pathogen, and Bacteroides fragilis were significantly enriched (adjusted p-values <0.001 in both cases) in F samples from CRC patients when compared to F samples from non-CRC individuals (Figure 3). In contrast, focusing on ASVs level analysis, Blautia sp. and Faecalibacterium sp. were significantly more abundant in F samples from the non-CRC control group (adjusted p-values of <0.05 and <0.01, respectively) (Figure 3).

When the analysis was focused only on oral related microorganisms, the genera Fusobacterium and Parvimonas appeared across all types of samples except in F of non-CRC individuals (Figure 4A), showing more abundance in GCF, Ac, NM and F of CRC patients (Figure 4A). Differential abundance analysis of specific combinations of ASVs integrating typical oral related genera was conducted using data obtained from F samples from CRC and non-CRC individuals. The data revealed that the group formed by Parvimonas, Fusobacterium, Prevotella, Peptostreptococcus and Porphyromonas was over-abundant in CRC compared to non-CRC fecal samples (Figure 4B). However, only Fusobacterium, Parvimonas and Peptostreptococcus showed significant differences when used alone (Figure 3).

In addition, the microbiome differences between the Ac and NM samples obtained from the same individuals with CRC were analyzed (Figure 2). At the genus level, Fusobacterium and Prevotella were over-represented in Ac samples compared to NM samples from the same individual. In contrast, the Ruminococcus gnavus and torques groups, Anaerostipes and Blautia were more abundant in NM than in Ac (Figure 2B). At the species level, the results demonstrated that Bacteroides fragilis was enriched in Ac tissue (8.7%) when compared to NM samples (5%) of CRC patients, in accordance with results observed in F samples of CRC patients when compared to samples from non-CRC individuals (Figure 2C).

No significant differences in alpha diversity among the CRC and non-CRC groups for F and S samples were detected. When quantifying the influence of covariables in beta-diversity measures, F appeared to be influenced by patient group (CRC, non-CRC; p-value <0.01) and age (p-value <0.05), but not sex. Analysis of Ac samples revealed significant influences of age (p-value <0.05) and sex (p-value <0.05), but not of the location (right vs. left colon), tumor size, metastasis, or lymph node affectation. Additionally, S samples were not influenced by patient group, age, or sex.

### Bacterial co-ocurrence

To corroborate the hypothesis that oral microbes translocate in complex multispecies clusters, a correlation study was conducted. Paired S, GCF, NM, and Ac samples from the same CRC individuals were used to study intra- and interniche bacterial correlations. In GCF samples from CRC patients, members of the red and orange Socransky complexes, well-known as late colonizers and periodontopathogens, including Fusobacterium, Parvimonas and Tannerella forsythia, were positively correlated with each other. Members of the green and purple complexes, known as early colonizers and health-associated, also clustered together (Figure 5A). Interestingly, in S samples of CRC patients, Fusobacterium periodonticum correlated positively with members or the red complex (i.e. Porphyromonas) as well as with other facultative anaerobes (i.e., Gemella) and to a lesser extent with aerobes (i.e., Rothia aeria) (Figure 5B).

When studying the correlations of oral species in NM samples from CRC patients, common patterns with S samples were detected. In this niche, proteolytic species from the orange complex (Fusobacterium, Parvimonas and Peptostreptococcus) and facultative anaerobes (Gemella, Granulicatella and Streptococcus) were positively correlated (Figure 6A, 7A). In the Ac samples, the same proteolytic species (Fusobacterium, Parvimonas and Peptostreptococcus) clustered together and had little or no correlation with facultative anaerobes (apart from Gemella), resembling the subgingival niche. It is worth mentioning that in tumors, the proteolytic oral cluster also correlated with intestinal microorganisms, such as unclassified Hungatella and Bacteroides fragilis (positively) or Agathobacter and Faecalibacterium (negatively) (Figure 6B, 7B). Therefore, oral proteolytic bacteria have a correlation pattern in the gut, which seems to be a pattern found in oral niches.

Finally, the correlation between bacteria in saliva or subgingival fluids and their presence in tumor tissue was analysed, and no strong correlations were found. In fact, only Gemella and Veillonella correlated positively with itself when comparing the Ac and S samples (Figure 8). Therefore, these results suggest that the levels of oral bacteria in the gut are not related to their corresponding levels in the oral cavity.

### Gut enterotypes and colonization of oral bacteria

To evaluate whether certain bacterial communities in the colon are more likely to interact or facilitate colonization by oral bacteria, we analyzed the distinct enterotypes in NM samples. The DMM algorithm identified the optimal number of enterotypes in the NM tissues as two based on the Laplace approximation and NMD ordination (Figure 9A). Although some overlapping was observed between the clusters, the differences among them were significant. Indeed, the canonical correlation analysis (CCA) performed for the two possible enterotypes in the control/affected tissue pairs showed a higher distance among the enterotypes than among the control/affected tissue pairs (Figure 9B). Patients classified in enterotype 1 or 2 did not differ in the clinical parameters evaluated (age, sex, tumor status, or location) (Table 3).

**Table 3. Clinical parameters of patient classified by tissue cluster (enterotype).**

| | | **Cluster-1** | **Cluster-2** | ***p*-value** |
|---|---|---|---|---|
| **Localization *** | **Right** | 14 | 11 | 0.15 |
| | **Left** | 9 | 13 | |
| **Tumor size** | **T1** | 2 | 4 | 0.19 |
| | **T2** | 4 | 2 | |
| | **T3** | 16 | 17 | |
| | **T4** | 1 | 0 | |
| **Spread to lymph nodes** | **N0** | 16 | 12 | 0.14 |
| | **N>0** | 7 | 12 | |
| **Metastasis** | **M0** | 21 | 23 | 0.45 |
| | **M>0** | 2 | 1 | |
| **Sex** | **Male** | 17 | 16 | 0.5 |
| | **Female** | 6 | 8 | |
| **Age** | **Mean** | 68.78 | 66.29 | 0.15 |

| | | | | |
|---|---|---|---|---|
| *The right colon refers to: the cecum, ascending colon, hepatic flexure and transverse colon. The left colon refers to: the splenic flexure, descending colon, sigmoid colon and rectum. | | | | |

Differences in the bacterial composition were evaluated according to the enterotypes assigned to the tumor tissues. A total of 39 species were found at significantly different levels between tumors of enterotype 1 and 2. Among them, only an uncultured Fusobacterium could potentially have an oral origin, and is not one of the most prevalent or abundant.

### Determination of the best bacterial consortium for CRC diagnostics

The role of Fusobacterium as a potential biomarker in fecal samples was tested in our cohort. ROC curves were utilized to assess its discriminatory power, revealing an AUC of 0.65 when only Fusobacterium was included in the model (Figure 10). Interestingly, the inclusion in the model of other over-abundant bacteria in CRC feces like Parvimonas (oral) and Bacteroides (intestinal), improved the discriminatory power between CRC patients and non-CRC individuals, obtaining an AUC of 0.75 (Figure 10). However, the addition of other over-abundant bacteria in CRC feces like Peptostreptoccocus did not increase the efficiency of the model, whereas the addition of healthy associated bacteria like Blautia or Faecalibacterium increased the AUC value up to 0.77 and 0.8, respectively. Feature selection using the Boruta algorithm confirmed the efficiency of Fusobacterium and Parvimonas as biomarkers for CRC. However, this algorithm suggested a combination of six other genera, namely, Incertae sedis, Odoribacter, Faecalitalea, UCG-010, Slackia and Parvimonas, which achieved an AUC of 0.86, although the relative proportions of most of these poorly characterized bacteria were very low in the samples. Therefore, the present invention provides new evidence that oral pathobionts, probably forming synergistic consortia, colonize the colorectal mucosa, and can be detected in fecal samples. The periodontal bacterial associations reported in the present work may enhance the development of a pro-inflammatory microenvironment, collaborating with the onset of tumorigenesis. In the present invention, we propose that the cluster formed by Fusobacterium, Parvimonas, Bacteroides and Faecalibacterium can be used as an excellent biomarker for early diagnosis of CRC. We also aimed to highlight that PD is a risk factor for CRC initiation. Therefore, oral treatments could contribute to reducing the incidence and prevalence of cancer; however, CRC screening for patients diagnosed with PD may be key to improving the early diagnosis of CRC.

### REFERENCES

1. International Agency for Research on Cancer (IARC), World Health Organization (WHO). Global Cancer Observatory (GLOBOCAN) 2020. Lyon (France): IARC; 2020 [accessed 2022 Dec 12]. https://gco.iarc.fr/.
2. Asociacion Española Contra el Cáncer. Informe dinámico: Cáncer de Colon. Madrid (Spain): Asociacion Española Contra el Cáncer; 2023 Jan 01 [accessed 2022 Sep 26]. https://observatorio.contraelcancer.es/informes/informe-dinamico-cancer-de-colon.
3. Kong F & Cai Y (2019) Study Insights into Gastrointestinal Cancer through the Gut Microbiota. BioMed Research International 2019, 8721503, doi: 10.1155/2019/8721503.
4. Louis P, Hold GL & Flint HJ (2014) The gut microbiota, bacterial metabolites and colorectal cancer. Nature Reviews Microbiology 12, 661-672, doi: 10.1038/nrmicro3344.
5. Salehiniya H, Pouyesh V, Tarazoj AA, Mohammadian-Hafshejani A, Aghajani M, Yousefi SM & Gandomani HS (2017) Colorectal cancer in the world: incidence, mortality and risk factors. Biomedical Research and Therapy 4, doi: 10.15419/bmrat.v4i10.372.
6. Sawicki T, Ruszkowska M, Danielewicz A, Niedźwiedzka E, Arlukowicz T & Przybylowicz KE (2021) A review of colorectal cancer in terms of epidemiology, risk factors, development, symptoms and diagnosis. Cancers 13, 2025, doi: 10.3390/cancers13092025.
7. Servizo Galego de Saúde (SERGAS). Programa de detection precoz do cancro colorrectal. Galicia (Spain): SERGAS;2013 [accessed 2022 Sep 28]. https://www.sergas.es/Saude-publica/Programa-de-detecci%C3%B3n-precoz--do-cancro-colorrectal?idioma=es.
8. Burnett-Hartman AN, Lee JK, Demb J & Gupta S (2021) An update on the epidemiology, molecular characterization, diagnosis, and screening strategies for early-onset colorectal cancer. Gastroenterology 160, 1041-1049, doi:10.1053/j.gastro.2020.12.068.
9. Siegel RL, Torre LA, Soerjomataram I, Hayes RB, Bray F, Weber TK & Jemal A (2019) Global patterns and trends in colorectal cancer incidence in young adults. Gut 68, 2179-2185, doi: 10.1136/gutjnl-2019-319511.
10. Vuik FE, Nieuwenburg SA, Bardou M, Lansdorp-Vogelaar I, Dinis-Ribeiro M, Bento MJ, Zadnik V, Pellisé M, Esteban L, Kaminski MF, Suchanek S, Ngo O, Májek O, Leja M, Kuipers EJ & Spaander MC (2019) Increasing incidence of colorectal cancer in young adults in Europe over the last 25 years. Gut 68, 1820-1826, doi: 10.1136/gutjnl-2018-317592.
11. Elsafi SH, Alqahtani NI, Zakary NY & Al Zahrani EM (2015) The sensitivity, specificity, predictive values, and likelihood ratios of fecal occult blood test for the detection of colorectal cancer in hospital settings. Clinical experimental gastroenterology, 279-284.
12. Watson AJ & Collins PD (2011) Colon cancer: a civilization disorder. Digestive Diseases 29, 222-228, doi: 10.1159/000323926.
13. Ahmad AF, Dwivedi G, O'Gara F, Caparros-Martin J & Ward NC (2019) The gut microbiome and cardiovascular disease: current knowledge and clinical potential. Am J Physiol Heart Circ Physiol 317, H923-H938, doi: 10.1152/ajpheart.00376.2019.
14. Chok KC, Ng KY, Koh RY & Chye SM (2021) Role of the gut microbiome in Alzheimer's disease. Reviews in the Neurosciences 32, 767-789, doi:10.1515/revneuro-2020-0122.
15. Cryan JF, O'Riordan KJ, Sandhu K, Peterson V & Dinan TG (2020) The gut microbiome in neurological disorders. The Lancet Neurology 19, 179-194, doi: 10.1016/S1474-4422(19)30356-4.
16. du Teil Espina M, Gabarrini G, Harmsen HJM, Westra J, van Winkelhoff AJ & van Dijl JM (2018) Talk to your gut: the oral-gut microbiome axis and its immunomodulatory role in the etiology of rheumatoid arthritis. FEMS microbiology reviews 43, 1-18, doi: 10.1093/femsre/fuy035
17. Durack J & Lynch SV (2018) The gut microbiome: Relationships with disease and opportunities for therapy. Journal of Experimental Medicine 216, 20-40, doi: 10.1084/jem.20180448
18. Gasmi Benahmed A, Gasmi A, Dosa A, Chirumbolo S, Mujawdiya PK, Aaseth J, Dadar M & Bjørklund G (2021) Association between the gut and oral microbiome with obesity. Anaerobe 70, 102248, doi: 10.1016/j.anaerobe.2020.102248.
19. Peirce JM & Alviña K (2019) The role of inflammation and the gut microbiome in depression and anxiety. Journal of Neuroscience Research 97, 1223-1241, doi: 10.1002/jnr.24476.
20. Stokholm J, Blaser MJ, Thorsen J, Rasmussen MA, Waage J, Vinding RK, Schoos A-MM, Kunøe A, Fink NR, Chawes BL, Bønnelykke K, Brejnrod AD, Mortensen MS, Al-Soud WA, Sørensen SJ & Bisgaard H (2018) Maturation of the gut microbiome and risk of asthma in childhood. Nature Communications 9, 141, doi: 10.1038/s41467-017-955 02573-2.
21. Mohammadi M, Mirzaei H & Motallebi M (2021) The role of anaerobic bacteria in the development and prevention of colorectal cancer: A review study. Anaerobe, 102501, doi: 10.1016/j.anaerobe.2021.102501.
22. Nejman D, Livyatan I, Fuks G, Gavert N, Zwang Y, Geller LT, Rotter-Maskowitz A, Weiser R, Mallel G, Gigi E, Meltser A, Douglas GM, Kamer I, Gopalakrishnan V, Dadosh T, Levin-Zaidman S, Avnet S, Atlan T, Cooper ZA, Arora R, Cogdill AP, Khan MAW, Ologun G, Bussi Y, Weinberger A, Lotan-Pompan M, Golani O, Perry G, Rokah M, Bahar-Shany K, Rozeman EA, Blank CU, Ronai A, Shaoul R, Amit A, Dorfman T, Kremer R, Cohen ZR, Harnof S, Siegal T, Yehuda-Shnaidman E, Gal-Yam EN, Shapira H, Baldini N, Langille MGI, Ben-Nun A, Kaufman B, Nissan A, Golan T, Dadiani M, Levanon K, Bar J, Yust-Katz S, Barshack I, Peeper DS, Raz DJ, Segal E, Wargo JA, Sandbank J, Shental N & Straussman R (2020) The human tumor microbiome is composed of tumor type-specific intracellular bacteria. Science (New York, NY) 368, 973-980, doi: 10.1 126/science.aay9189.
23. Osman MA, Neoh HM, Ab Mutalib NS, Chin SF, Mazlan L, Raja Ali RA, Zakaria AD, Ngiu CS, Ang MY & Jamal R (2021) Parvimonas micra, Peptostreptococcus stomatis, Fusobacterium nucleatum and Akkermansia muciniphila as a four-bacteria biomarker panel of colorectal cancer. Scientific Reports 11, 2925, doi: 10.1038/s41598-021-82465-0.
24. Parhi L, Alon-Maimon T, Sol A, Nejman D, Shhadeh A, Fainsod-Levi T, Yajuk O, Isaacson B, Abed J, Maalouf N, Nissan A, Sandbank J, Yehuda-Shnaidman E, Ponath F, Vogel J, Mandelboim O, Granot Z, Straussman R & Bachrach G (2020) Breast cancer colonization by Fusobacterium nucleatum accelerates tumor growth and metastatic progression. Nature Communications 11, 3259, doi: 10.1038/s41467-020-16967-2.
25. Purcell RV, Visnovska M, Biggs PJ, Schmeier S & Frizelle FA (2017) Distinct gut microbiome patterns associate with consensus molecular subtypes of colorectal cancer. Scientific Reports 7, 11590, doi: 10.1038/s41598-017-11237-6.
26. Wong SH & Yu J (2019) Gut microbiota in colorectal cancer: mechanisms of action and clinical applications. Nature Reviews Gastroenterology & Hepatology 16, 690-, doi: 10.1038/s41575-019-0209-8.
27. Schmidt TSB, Raes J & Bork P (2018) The Human Gut Microbiome: From Association to Modulation. Cell 172, 1198-1215, doi: 10.1016/j.cell.2018.02.044.
28. Wong SH & Yu J (2019) Gut microbiota in colorectal cancer: mechanisms of action and clinical applications. Nature Reviews Gastroenterology & Hepatology 16, 690-704, doi: 10.1038/s41575-019-0209-8.
29. Yang Y, Nguyen M, Khetrapal V, Sonnert ND, Martin AL, Chen H, Kriegel MA & Palm NW (2022) Within-host evolution of a gut pathobiont facilitates liver translocation. Nature, doi: 10.1038/s41586-022-04949-x.
30. Bertocchi A, Carloni S, Ravenda PS, Bertalot G, Spadoni I, Lo Cascio A, Gandini S, Lizier M, Braga D, Asnicar F, Segata N, Klaver C, Brescia P, Rossi E, Anselmo A, Guglietta S, Maroli A, Spaggiari P, Tarazona N, Cervantes A, Marsoni S, Lazzari L, Jodice MG, Luise C, Erreni M, Pece S, Di Fiore PP, Viale G, Spinelli A, Pozzi C, Penna G & Rescigno M (2021) Gut vascular barrier impairment leads to intestinal bacteria dissemination and colorectal cancer metastasis to liver. Cancer Cell 39, 708-724 e711, doi: 10.1016/j.ccell.2021.03.004.
31. Koliarakis I, Messaritakis I, Nikolouzakis TK, Hamilos G, Souglakos J & Tsiaoussis J (2019) Oral bacteria and intestinal dysbiosis in colorectal cancer. International Journal of Molecular Sciences 20, 4146.
32. Xu J, Yang M, Wang D, Zhang S, Van S, Zhu Y & Chen W (2020) Alteration of the abundance of Parvimonas micra in the gut along the adenoma-carcinoma sequence. Oncology Letters 20, 106, doi: 10.3892/ol.2020.11967.
33. Akimoto N, Ugai T, Zhong R, Hamada T, Fujiyoshi K, Giannakis M, Wu K, Cao Y, Ng K & Ogino S (2021) Rising incidence of early-onset colorectal cancer - a call to action. Nature Reviews Clinical Oncology 18, 230-243, doi: 10.1038/s41571-020-00445-1.
34. Hofseth LJ, Hebert JR, Chanda A, Chen H, Love BL, Pena MM, Murphy EA, Sajish M, Sheth A, Buckhaults PJ & Berger FG (2020) Early-onset colorectal cancer: initial clues and current views. Nature Reviews Gastroenterology & Hepatology 17, 352-364, doi: 10.1038/s41575-019-0253-4.
35. Abed J, Maalouf N, Manson AL, Earl AM, Parhi L, Emgard JEM, Klutstein M, Tayeb S, Almogy G, Atlan KA, Chaushu S, Israeli E, Mandelboim O, Garrett WS & Bachrach G (2020) Colon cancer-associated Fusobacterium nucleatum may originate from the oral cavity and reach colon tumors via the circulatory system. Frontiers in Cellular and Infection Microbiology 10, 400, doi: 10.3389/fcimb.2020.00400.
36. Flemer B, Warren RD, Barrett MP, Cisek K, Das A, Jeffery IB, Hurley E, O'Riordain M, Shanahan F & O'Toole PW (2018) The oral microbiota in colorectal cancer is distinctive and predictive. Gut 67, 1454-1463, doi: 10.1136/gutjnl-2017-314814.
37. Mesa F, Mesa-Lopez MJ, Egea-Valenzuela J, Benavides-Reyes C, Nibali L, Ide M, Mainas G, Rizzo M & Magan-Femandez A (2022) A new comorbidity in periodontitis: Fusobacterium nucleatum and colorectal cancer. Medicine 58, doi: 10.3390/medicina58040546.
38. Wang N & Fang JY (2022) Fusobacterium nucleatum, a key pathogenic factor and microbial biomarker for colorectal cancer. Trends in microbiology, doi: 10.1016/j.tim.2022.08.010.
39. Brentar M, Kuijvenhoven J, Stokkers P & Struys E (2022) The potential of fecal microbiota and amino acids to detect and monitor patients with adenoma. Gut microbes 14, 2038863.
40. Clos-Garcia M, Garcia K, Alonso C, Iruarrizaga-Lejarreta M, D'Amato M, Crespo A, Iglesias A, Cubiella J, Bujanda L & Falcón-Pérez JM (2020) Integrative analysis of fecal metagenomics and metabolomics in colorectal cancer. Cancers 12, 1142.
41. Eklöf V, Löfgren-Burström A, Zingmark C, Edin S, Larsson P, Karling P, Alexeyev O, Rutegård J, Wikberg ML & Palmqvist R (2017) Cancer-associated fecal microbial markers in colorectal cancer detection. International journal of cancer 141, 2528-2536.
42. Goedert JJ, Gong Y, Hua X, Zhong H, He Y, Peng P, Yu G, Wang W, Ravel J & Shi J (2015) Fecal microbiota characteristics of patients with colorectal adenoma detected by screening: a population-based study. EBioMedicine 2, 597-603.
43. Tarallo S, Ferrero G, Gallo G, Francavilla A, Clerico G, Realis Luc A, Manghi P, Thomas AM, Vineis P & Segata N (2019) Altered fecal small RNA profiles in colorectal cancer reflect gut microbiome composition in stool samples. Msystems 4, e00289-00219.
44. Yachida S, Mizutani S, Shiroma H, Shiba S, Nakajima T, Sakamoto T, Watanabe H, Masuda K, Nishimoto Y, Kubo M, Hosoda F, Rokutan H, Matsumoto M, Takamaru H, Yamada M, Matsuda T, Iwasaki M, Yamaji T, Yachida T, Soga T, Kurokawa K, Toyoda A, Ogura Y, Hayashi T, Hatakeyama M, Nakagama H, Saito Y, Fukuda S, Shibata T & Yamada T (2019) Metagenomic and metabolomic analyses reveal distinct stage-specific phenotypes of the gut microbiota in colorectal cancer. Nature Medicine 25, 968-976, doi: 10.1038/s41591-019-0458-7.
45. Young C, Wood HM, Fuentes Balaguer A, Bottomley D, Gallop N, Wilkinson L, Benton SC, Brealey M, John C & Burtonwood C (2021) Microbiome analysis of more than 2,000 nhs bowel cancer screening programme samples shows the potential to improve screening accuracymicrobiome analysis improves crc screening accuracy. Clinical Cancer Research 27, 2246-2254.
46. Zeller G, Tap J, Voigt AY, Sunagawa S, Kultima JR, Costea PI, Amiot A, Böhm J, Brunetti F & Habermann N (2014) Potential of fecal microbiota for early-stage detection of colorectal cancer. Molecular Systems Biology 10, 766.
47. Drewes JL, White JR, Dejea CM, Fathi P, Iyadorai T, Vadivelu J, Roslani AC, Wick EC, Mongodin EF, Loke MF, Thulasi K, Gan HM, Goh KL, Chong HY, Kumar S, Wanyiri JW & Sears CL (2017) High-resolution bacterial 16S rRNA gene profile meta-analysis and biofilm status reveal common colorectal cancer consortia. NPJ Biofilms Microbiomes 3, 34,doi: 10.1038/s41522-017-0040-3.
48. Mangifesta M, Mancabelli L, Milani C, Gaiani F, de'Angelis N, de'Angelis GL, van Sinderen D, Ventura M & Turroni F (2018) Mucosal microbiota of intestinal polyps reveals putative biomarkers of colorectal cancer. Scientific Reports 8, 13974, doi: 10.1038/s41598-018-32413-2.
49. Wang Y, Zhang Y, Qian Y, Xie Y-H, Jiang S-S, Kang Z-R, Chen Y-X, Chen Z-F & Fang J-Y (2021) Alterations in the oral and gut microbiome of colorectal cancer patients and association with host clinical factors. 149, 925-935, doi: 10.1002/ijc.33596.
50. Zhang M, Lv Y, Hou S, Liu Y, Wang Y & Wan X (2021) Differential mucosal microbiome profiles across stages of human colorectal cancer. Life 11, 831.
51. Löwenmark T, Löfgren-Burström A, Zingmark C, Ekl6f V, Dahlberg M, Wai S, Larsson P, Ljuslinder I, Edin S & Palmqvist R (2020) Parvimonas micra as a putative non-invasive faecal biomarker for colorectal cancer. Scientific Reports 10, 15250, doi: 10.1038/s41598-020-72132-1.
52. Komiya Y, Shimomura Y, Higurashi T, Sugi Y, Arimoto J, Umezawa S, Uchiyama S, Matsumoto M & Nakajima A (2019) Patients with colorectal cancer have identical strains of Fusobacterium nucleatum in their colorectal cancer and oral cavity. Gut 68, 1335-1337, doi: 10.1136/gutjnl-2018-316661.
53. Xuan K, Jha AR, Zhao T, Uy JP & Sun C (2021) Is periodontal disease associated with increased risk of colorectal cancer? A meta-analysis. International Journal of Dental Hygiene 19, 50-61, doi: 10.1111/idh.12483.
54. Papapanou PN, Sanz M, Buduneli N, Dietrich T, Feres M, Fine DH, Flemmig TF, Garcia R, Giannobile WV, Graziani F, Greenwell H, Herrera D, Kao RT, Kebschull M, Kinane DF, Kirkwood KL, Kocher T, Kornman KS, Kumar PS, Loos BG, Machtei E, Meng H, Mombelli A, Needleman I, Offenbacher S, Seymour GJ, Teles R & Tonetti MS (2018) Periodontitis: Consensus report of workgroup 2 of the 2017 World Workshop on the Classification of Periodontal and Peri-Implant Diseases and Conditions. Journal of clinical periodontology 45 Suppl 20, S162-s170, doi: 10.1111/jcpe.12946.
55. Löe H (1967) The Gingival Index, the Plaque Index and the Retention Index Systems. Journal of periodontology 38, Suppl:610-616, doi: 10.1902/jop.1967.38.6.610.
56. Klindworth A, Pruesse E, Schweer T, Peplies J, Quast C, Horn M & Glöckner FO (2013) Evaluation of general 16S ribosomal RNA gene PCR primers for classical and next-generation sequencing-based diversity studies. Nucleic acids research 41, e1, doi: 10.1093/nar/gks808.
57. Andrews S. FastQC. California (USA): GitHub Inc, 2023 Apr 25. [accessed 2023 Jun 10]. https://github.com/s-andrews/FastQC.
58. Bolyen E, Rideout JR, Dillon MR, Bokulich NA, Abnet CC, Al-Ghalith GA, 1103 Alexander H, Alm EJ, Arumugam M, Asnicar F, Bai Y, Bisanz JE, Bittinger K, Brejnrod A, Brislawn CJ, Brown CT, Callahan BJ, Caraballo-Rodriguez AM, Chase J, Cope EK, Da Silva R, Diener C, Dorrestein PC, Douglas GM, Durall DM, Duvallet C, Edwardson CF, Ernst M, Estaki M, Fouquier J, Gauglitz JM, Gibbons SM, Gibson DL, Gonzalez A, Gorlick K, Guo J, Hillmann B, Holmes S, Holste H, Huttenhower C, Huttley GA, Janssen S, Jarmusch AK, Jiang L, Kaehler BD, Kang KB, Keefe CR, Keim P, Kelley ST, Knights D, Koester I, Kosciolek T, Kreps J, Langille MGI, Lee J, Ley R, Liu Y-X, Loftfield E, Lozupone C, Maher M, Marotz C, Martin BD, McDonald D, McIver LJ, Melnik AV, Metcalf JL, Morgan SC, Morton JT, Naimey AT, Navas-Molina JA, Nothias LF, Orchanian SB, Pearson T, Peoples SL, Petras D, Preuss ML, Pruesse E, Rasmussen LB, Rivers A, Robeson MS, Rosenthal P, Segata N, Shaffer M, Shiffer A, Sinha R, Song SJ, Spear JR, Swafford AD, Thompson LR, Torres PJ, Trinh P, Tripathi A, Tumbaugh PJ, Ul-Hasan S, van der Hooft JJJ, Vargas F, Vázquez-Baeza Y, Vogtmann E, von Hippel M, Walters W, Wan Y, Wang M, Warren J, Weber KC, Williamson CHD, Willis AD, Xu ZZ, Zaneveld JR, Zhang Y, Zhu Q, Knight R & Caporaso JG (2019) Reproducible, interactive, scalable and extensible microbiome data science using QIIME 2. Nature Biotechnology 37, 852-857, doi: 10.1038/s41587-019-0209-9.
59. Callahan BJ, McMurdie PJ, Rosen MJ, Han AW, Johnson AJA & Holmes SP (2016) DADA2: High-resolution sample inference from Illumina amplicon data. Nature Methods 13, 581-583, doi: 10.1038/nmeth.3869.
60. Quast C, Pruesse E, Yilmaz P, Gerken J, Schweer T, Yarza P, Peplies J & Glöckner FO (2013) The SILVA ribosomal RNA gene database project: improved data processing and web-based tools. Nucleic acids research 41, D590-596, doi:10.1093/nar/gks1219.
61. Ihaka R & Gentleman R (1996) R: A Language for Data Analysis and Graphics. Journal of Computational and Graphical Statistics 5, 299-314, doi: 10.1080/10618600.1996.10474713.
62. McMurdie PJ & Holmes S (2013) phyloseq: an R package for reproducible interactive analysis and graphics of microbiome census data. PloS one 8, e61217, doi: 10.1371/journal.pone.0061217.
63. Salter SJ, Cox MJ, Turek EM, Calus ST, Cookson WO, Moffatt MF, Turner P, Parkhill J, Loman NJ & Walker AW (2014) Reagent and laboratory contamination can critically impact sequence-based microbiome analyses. BMC Biology 12, 87, doi: 10.1186/s12915-014-0087-z.
64. Lin H & Peddada SD (2020) Analysis of compositions of microbiomes with bias correction. Nature Communications 11, 3514, doi: 10.1038/s41467-020-17041-7.
65. Holm S (1979) A simple sequentially rejective multiple test procedure. Scandinavian Journal of Statistics 6, 65-70.
66. Rohart F, Gautier B, Singh A & Lê Cao K-A (2017) mixOmics: An R package for 'omics feature selection and multiple data integration. PLoS computational biology 13, e1005752, doi: 0.1371/journal.pcbi.1005752.
67. Holmes I, Harris K & Quince C (2012) Dirichlet multinomial mixtures: generative models for microbial metagenomics. PloS one 7, e30126.
68. Oksanen J SG, Blanchet F, Kindt R, Legendre P, Minchin P, O'Hara R, Solymos P, Stevens M, Szoecs E, Wagner H, Barbour M, Bedward M, Bolker B, Borcard D, Carvalho G, Chirico M, De Caceres M, Durand S, Evangelista H, FitzJohn R, Friendly M, Furneaux B, Hannigan G, Hill M, Lahti L, McGlinn D, Ouellette M, Ribeiro Cunha E, Smith T, Stier A, Ter Braak C, Weedon J (2023) vegan: Community Ecology Package.
69. Mattiuzzi C, Sanchis-Gomar F & Lippi G (2019) Concise update on colorectal cancer epidemiology. Annals of translational medicine 7, 609, doi:10.21037/atm.2019.07.91.
70. Fan X, Jin Y, Chen G, Ma X & Zhang L (2021) Gut microbiota dysbiosis drives the development of colorectal cancer. Digestion 102, 508-515, doi: 10.1159/000508328.
71. Conde-Pérez K, Buetas E, Aja-Macaya P, Arribas E, Iglesias-Corrás I, Trigo-Tasende N, Nasser-Ali M, Estévez LS, Rumbo-Feal S & Otero-Alén B (2022) Parvimonas micra can translocate from the subgingival sulcus of the human oral cavity to colorectal adenocarcinoma. Molecular Oncology, accepted author manuscript, doi:10.1002/1878-0261.13506.
72. Jin S, Wetzel D & Schirmer M (2022) Deciphering mechanisms and implications of bacterial translocation in human health and disease. Current Opinion in Microbiology 67, 102147.
73. Yu TC, Zhou YL, Fang JYJJoG & Hepatology (2022) Oral pathogen in the pathogenesis of colorectal cancer. 37, 273-279.
74. Shen X, Li J, Li J, Zhang Y, Li X, Cui Y, Gao Q, Chen X, Chen Y & Fang JY (2021) Fecal enterotoxigenic Bacteroides fragilis-Peptostreptococcus stomatis-Parvimonas micra biomarker for noninvasive diagnosis and prognosis of colorectal laterally spreading tumor. Frontiers in Oncology 11, 661048, doi:10.3389/fonc.2021.661048.
75. Ang MY, Dymock D, Tan JL, Thong MH, Tan QK, Wong GJ, Paterson IC & Choo SW (2013) Genome Sequence of Parvimonas micra Strain A293, Isolated from an Abdominal Abscess from a Patient in the United Kingdom. 1, e01025-01013, doi: doi:10.1128/genomeA.01025-13.
76. Hu L, Liu Y, Kong X, Wu R, Peng Q, Zhang Y, Zhou L & Duan L (2021) Fusobacterium nucleatum facilitates M2 macrophage polarization and colorectal carcinoma progression by activating TLR4/NF-κB/S100A9 cascade. Front Immunol 12, 658681, doi: 10.3389/fimmu.2021.658681.
77. Long X, Wong CC, Tong L, Chu ESH, Ho Szeto C, Go MYY, Coker OO, Chan AWH, Chan FKL, Sung JJY & Yu J (2019) Peptostreptococcus anaerobius promotes colorectal carcinogenesis and modulates tumour immunity. Nature Microbiology 4, 2319-2330, doi: 10.1038/s41564-019-0541-3.
78. Lowenmark T, Lofgren-Burstrom A, Zingmark C, Ljuslinder I, Dahlberg M, Edin S & Palmqvist R (2022) Tumour colonisation of Parvimonas micra is associated with decreased survival in colorectal cancer patients. Cancers 14, doi: 1 0.3390/cancers 14235937.
79. Mu W, Jia Y, Chen X, Li H, Wang Z, Cheng BJFiC & Microbiology I (2020) Intracellular Porphyromonas gingivalis promotes the proliferation of colorectal cancer cells via the MAPK/ERK signaling pathway. 10, 584798.
80. Van Dalen PJ, Van Deutekom-Mulder EC, De Graaff J & Van Steenbergen TJ (1998) Pathogenicity of Peptostreptococcus micros morphotypes and Prevotella species in pure and mixed culture. Journal of medical microbiology 47, 135-140, doi: 10.1099/00222615-47-2-135.
81. Wang X, Jia Y, Wen L, Mu W, Wu X, Liu T, Liu X, Fang J, Luan Y, Chen P, Gao J, Nguyen K-A, Cui J, Zeng G, Lan P, Chen Q, Cheng B & Wang Z (2021) Porphyromonas gingivalis promotes colorectal carcinoma by activating the hematopoietic NLRP3 inflammasome. Cancer research 81, 2745-2759, doi:10.1158/0008-5472.CAN-20-3827 %J Cancer Research.
82. Zhao L, Zhang X, Zhou Y, Fu K, Lau HC-H, Chun TW-Y, Cheung AH-K, Coker OO, Wei H, Wu WK-K, Wong SH, Sung JJ-Y, To KF & Yu J (2022) Parvimonas micra promotes colorectal tumorigenesis and is associated with prognosis of colorectal cancer patients. Oncogene, doi: 10.1038/s41388-022-02395-7.
83. Castellarin M, Warren RL, Freeman JD, Dreolini L, Krzywinski M, Strauss J, Barnes R, Watson P, Allen-Vercoe E, Moore RA & Holt RA (2012) Fusobacterium nucleatum infection is prevalent in human colorectal carcinoma. Genome Res 22, 299-306, doi: 10.1101/gr. 126516.111.
84. Hertel J, Heinken A, Martinelli F & Thiele I (2021) Integration of constraint-based modeling with fecal metabolomics reveals large deleterious effects of Fusobacterium spp. on community butyrate production. Gut microbes 13, 1-23, doi: 10.1080/19490976.2021.1915673.
85. Kostic AD, Gevers D, Pedamallu CS, Michaud M, Duke F, Earl AM, Ojesina AI, Jung J, Bass AJ, Tabernero J, Baselga J, Liu C, Shivdasani RA, Ogino S, Birren BW, Huttenhower C, Garrett WS & Meyerson M (2012) Genomic analysis identifies association of Fusobacterium with colorectal carcinoma. Genome Res 22, 292-298, doi:10.1101/gr.126573.111.
86. Mima K, Nishihara R, Qian ZR, Cao Y, Sukawa Y, Nowak JA, Yang J, Dou R, Masugi Y & Song M (2016) Fusobacterium nucleatum in colorectal carcinoma tissue and patient prognosis. Gut 65, 1973-1980.
87. Rubinstein MR, Wang X, Liu W, Hao Y, Cai G & Han YW (2013) Fusobacterium nucleatum promotes colorectal carcinogenesis by modulating E-cadherin/β-catenin signaling via its FadA adhesin. Cell host & microbe 14, 195-206, doi:10.1016/j.chom.2013.07.012.
88. Serna G, Ruiz-Pace F, Hernando J, Alonso L, Fasani R, Landolfi S, Comas R, Jimenez J, Elez E, Bullman S, Tabernero J, Capdevila J, Dienstmann R & Nuciforo P (2020) Fusobacterium nucleatum persistence and risk of recurrence after preoperative treatment in locally advanced rectal cancer. Annals of oncology: official journal of the European Society for Medical Oncology 31, 1366-1375, doi: 10.1016/j.annonc.2020.06.003.
89. Tahara T, Yamamoto E, Suzuki H, Maruyama R, Chung W, Garriga J, Jelinek J, Yamano HO, Sugai T, An B, Shureiqi I, Toyota M, Kondo Y, Estécio MR & Issa JP (2014) Fusobacterium in colonic flora and molecular features of colorectal carcinoma. Cancer research 74, 1311-1318, doi: 10.1158/0008-5472.Can-13-1865.
90. Xu C, Fan L, Lin Y, Shen W, Qi Y, Zhang Y, Chen Z, Wang L, Long Y, Hou T, Si J & Chen S (2021) Fusobacterium nucleatum promotes colorectal cancer metastasis through miR-1322/CCL20 axis and M2 polarization. Gut microbes 13, 1980347, doi:10.1080/19490976.2021.1980347.
91. Yu T, Guo F, Yu Y, Sun T, Ma D, Han J, Qian Y, Kryczek I, Sun D, Nagarsheth N, Chen Y, Chen H, Hong J, Zou W & Fang JY (2017) Fusobacterium nucleatum Promotes Chemoresistance to Colorectal Cancer by Modulating Autophagy. Cell 170, 548-563.e516, doi:10.1016/j.cell.2017.07.008.
92. Nakatsu G, Li X, Zhou H, Sheng J, Wong SH, Wu WK, Ng SC, Tsoi H, Dong Y, Zhang N, He Y, Kang Q, Cao L, Wang K, Zhang J, Liang Q, Yu J & Sung JJ (2015) Gut mucosal microbiome across stages of colorectal carcinogenesis. Nat Commun 6, 8727, doi: 10.1038/ncomms9727.
93. Yu J, Feng Q, Wong SH, Zhang D, Liang Qy, Qin Y, Tang L, Zhao H, Stenvang J, Li Y, Wang X, Xu X, Chen N, Wu WKK, Al-Aama J, Nielsen HJ, Kiilerich P, Jensen BAH, Yau TO, Lan Z, Jia H, Li J, Xiao L, Lam TYT, Ng SC, Cheng AS-L, Wong VW-S, Chan FKL, Xu X, Yang H, Madsen L, Datz C, Tilg H, Wang J, Brünner N, Kristiansen K, Arumugam M, Sung JJ-Y & Wang J (2017) Metagenomic analysis of faecal microbiome as a tool towards targeted non-invasive biomarkers for colorectal cancer. Gut 66, 70-78, doi: 10.1136/gutjnl-2015-309800 %J Gut.
94. Boleij A, Hechenbleikner EM, Goodwin AC, Badani R, Stein EM, Lazarev MG, Ellis B, Carroll KC, Albesiano E, Wick EC, Platz EA, Pardoll DM & Sears CL (2015)The Bacteroides fragilis toxin gene is prevalent in the colon mucosa of colorectal cancer patients. Clinical infectious diseases : an official publication of the Infectious Diseases Society of America 60, 208-215, doi: 10.1093/cid/ciu787.
95. Bachem A, Makhlouf C, Binger KJ, de Souza DP, Tull D, Hochheiser K, Whitney PG, Fernandez-Ruiz D, Dähling S, Kastenmüller W, Jönsson J, Gressier E, Lew AM, Perdomo C, Kupz A, Figgett W, Mackay F, Oleshansky M, Russ BE, Parish IA, Kallies A, McConville MJ, Turner SJ, Gebhardt T & Bedoui S (2019) Microbiota-derived short-chain fatty acids promote the memory potential of antigen-activated CD8+ T cells. Immunity 51, 285-297.e285, doi: 10.1016/j.immuni.2019.06.002.
96. Liu X, Mao B, Gu J, Wu J, Cui S, Wang G, Zhao J, Zhang H & Chen W (2021) Blautia-a new functional genus with potential probiotic properties? Gut microbes 13, 1875796, doi: 10.1080/19490976.2021.1875796.
97. Luu K, Ye JY, Lagishetty V, Liang F, Hauer M, Sedighian F, Kwaan MR, Kazanjian KK, Hecht JR, Lin AY & Jacobs JP (2023) Fecal and tissue microbiota are associated with tumor T-cell infiltration and mesenteric lymph node involvement in colorectal cancer. Nutrients 15, 316.
98. Luu M, Riester Z, Baldrich A, Reichardt N, Yuille S, Busetti A, Klein M, Wempe A, Leister H, Raifer H, Picard F, Muhammad K, Ohl K, Romero R, Fischer F, Bauer CA, Huber M, Gress TM, Lauth M, Danhof S, Bopp T, Nerreter T, Mulder IE, Steinhoff U, Hudecek M & Visekruna A (2021) Microbial short-chain fatty acids modulate CD8+ T cell responses and improve adoptive immunotherapy for cancer. Nature Communications 12, 4077, doi: 10.1038/s41467-021-24331-1.
99. Chen L, Wang W, Zhou R, Ng SC, Li J, Huang M, Zhou F, Wang X, Shen B, M AK, Wu K & Xia B (2014) Characteristics of fecal and mucosa-associated microbiota inChinese patients with inflammatory bowel disease. Medicine 93, e51, doi:10.1097/md.0000000000000051.
100. Kim CH, Park J & Kim M (2014) Gut microbiota-derived short-chain Fatty acids, T cells, and inflammation. Immune network 14, 277-288, doi: 10.41 10/in.2014.14.6.277.
101. Maioli TU, Borras-Nogues E, Torres L, Barbosa SC, Martins VD, Langella P, Azevedo VA & Chatel JM (2021) Possible benefits of faecalibacterium prausnitzii for obesity-associated gut disorders. Frontiers in pharmacology 12, 740636, doi:10.3389/fphar.2021.740636.
102. Miquel S, Martin R, Rossi O, Bermúdez-Humarán LG, Chatel JM, Sokol H, Thomas M, Wells JM & Langella P (2013) Faecalibacterium prausnitzii and human intestinal health. Current Opinion in Microbiology 16, 255-261, doi:10.1016/j.mib.2013.06.003.
103. Zhang J, He Y, Xia L, Yi J, Wang Z, Zhao Y, Song X, Li J, Liu H & Liang X (2022) Expansion of colorectal cancer biomarkers based on gut bacteria and viruses. Cancers 14, 4662, doi: 10.3390/cancers14194662.
104. España CGdCdDd, 2022. Atlas de Salud Bucodental en España. Una llamada a la action., in: Comunicación GId (Ed.).
105. Simon-Soro A, Ren Z, Krom BP, Hoogenkamp MA, Cabello-Yeves PJ, Daniel SG, Bittinger K, Tomas I, Koo H & Mira A (2022) Polymicrobial aggregates in human saliva build the oral biofilm. mBio 13, e0013122, doi: 10.1128/mbio.00131-22.
106. Kolenbrander PE, Palmer Jr RJ, Rickard AH, Jakubovics NS, Chalmers NI & Diaz PI (2006) Bacterial interactions and successions during plaque development. Periodontology 2000 42, 47-79, doi: 10.1111/j.1600-0757.2006.00187.x.
107. Kolenbrander PE, Palmer RJ, Periasamy S & Jakubovics NS (2010) Oral multispecies biofilm development and the key role of cell-cell distance. Nature Reviews Microbiology 8, 471-480, doi: 10.1038/nrmicro2381.
108. Flynn KJ, Baxter NT & Schloss PD (2016) Metabolic and community synergy of oral bacteria in colorectal cancer. mSphere 1, doi: 10.1128/mSphere.00102-16.
109. Galeano Niño JL, Wu H, LaCourse KD, Kempchinsky AG, Baryiames A, Barber B, Futran N, Houlton J, Sather C & Sicinska E (2022) Effect of the intratumoral microbiota on spatial and cellular heterogeneity in cancer. Nature 611, 810-817.
110. El-Awady A, de Sousa Rabelo M, Meghil MM, Rajendran M, Elashiry M, Stadler AF, Foz AM, Susin C, Romito GA, Arce RM & Cutler CW (2019) Polymicrobial synergy within oral biofilm promotes invasion of dendritic cells and survival of consortia members. npj Biofilms and Microbiomes 5, 11, doi: 10.1038/s41522-019-0084-7.
111. Farrugia C, Stafford GP, Gains AF, Cutts AR & Murdoch C (2022) Fusobacterium nucleatum mediates endothelial damage and increased permeability following single species and polymicrobial infection. 93, 1421-1433, doi: 10.1002/JPER21-0671.
112. Socransky S, Haffajee A, Cugini M, Smith C & Kent Jr R (1998) Microbial complexes in subgingival plaque. Journal of clinical periodontology 25, 134-144, doi:10.1111/j.1600-051x.1998.tb02419.x.
113. Mark Welch JL, Rossetti BJ, Rieken CW, Dewhirst FE & Borisy GG (2016) Biogeography of a human oral microbiome at the micron scale. Proceedings of the National Academy of Sciences 113, E791-E800, doi: 10.1073/pnas.1522149113.
114. Bradshaw DJ, Marsh PD, Allison C & Schilling KM (1996) Effect of oxygen, inoculum composition and flow rate on development of mixed-culture oral biofilms. Microbiology 142, 623-629.
115. Rawat PS, Li Y, Zhang W, Meng X & Liu W (2022) Hungatella hathewayi, an efficient glycosaminoglycan-degrading Firmicutes from human gut and Its chondroitin ABC exolyase with high activity and broad substrate specificity. Applied Environmental Microbiology 88, e01546-01522, doi: 10.1128/aem.01546-22.
116. Mori M, Ponce-de-León M, Peretó J & Montero F (2016) Metabolic complementation in bacterial communities: necessary conditions and optimality. Frontiers in microbiology 7, 1553, doi: 10.3389/fmicb.2016.01553.
117. Sears CL & Pardoll DM (2011) Perspective: alpha-bugs, their microbial partners, and the link to colon cancer. Journal of Infectious Diseases 203, 306-311, doi:10.1093/jinfdis/jiq061.
118. Mirzaei R, Mirzaei H, Alikhani MY, Sholeh M, Arabestani MR, Saidijam M, Karampoor S, Ahmadyousefi Y, Moghadam MS, Irajian GR, Hasanvand H & Yousefimashouf R (2020) Bacterial biofilm in colorectal cancer: What is the real mechanism of action? Microbial pathogenesis 142, 104052, doi:10.1016/j.micpath.2020.104052.
119. Parsaei M, Sarafraz N, Moaddab SY & Ebrahimzadeh Leylabadlo H (2021) The importance of Faecalibacterium prausnitzii in human health and diseases. New microbes and new infections 43, 100928, doi: 10.1016/j.nmni.2021.100928.
120. Rosero JA, Killer J, Sechovcová H, Mrázek J, Benada O, Fliegerová K, Havlik J & Kopečný J (2016) Reclassification of Eubacterium rectale (Hauduroy et al. 1937) Prévot 1938 in a new genus Agathobacter gen. Nov. As agathobacter rectalis comb. Nov., and description of Agathobacter ruminis sp. Nov., isolated from the rumen contents of sheep and cows. International journal of systematic and evolutionary microbiology 66, 768-773, doi: 10.1099/ijsem.0.000788.
121. Mirzaei R, Afaghi A, Babakhani S, Sohrabi MR, Hosseini-Fard SR, Babolhavaeji K, Khani Ali Akbari S, Yousefimashouf R & Karampoor S (2021) Role of microbiota-derived short-chain fatty acids in cancer development and prevention. Biomedicine & Pharmacotherapy 139, 111619, doi: 10.1016/j.biopha.2021.111619.
122. Lv M, Zhang J, Deng J, Hu J, Zhong Q, Su M, Lin D, Xu T, Bai X, Li J & Guo X (2023) Analysis of the relationship between the gut microbiota enterotypes and colorectal adenoma. Frontiers in microbiology 14, Original Research, doi: 10.3389/fmicb.2023.1097892.
123. Zhao R, Xia D, Chen Y, Kai Z, Ruan F, Xia C, Gong J, Wu J & Wang X (2023) Improved diagnosis of colorectal cancer using combined biomarkers including Fusobacterium nucleatum, fecal occult blood, transferrin, CEA, CA19-9, gender, and age. Cancer medicine 12, 14636-14645, doi: 10.1002/cam4.6067.

## Claims

1. An *in vitro* method for determining the risk that a subject has colorectal cancer, comprising the following steps:
a. determining the levels or the concentration of bacteria belonging to the genus *Fusobacterium, Parvimonas, Bacteroides and*/*or Faecalibacterium,* or any combination thereof, in an intestinal or stool sample isolated from said subject; and
b. comparing the levels or the concentration of said bacteria in said intestinal or stool sample with one or more reference values, wherein an increase in the number of sequences of *Fusobacterium, Parvimonas, and*/*or Bacteroides,* and a decreased in the number of sequences of *Faecalibacterium,* in said sample with respect to said one or more reference values is indicative of an increase in the risk that the subject has colorectal cancer;
wherein the levels or the concentration of bacteria belonging to the genus *Fusobacterium, Parvimonas, Bacteroides and*/*or Faecalibacterium,* are determined by performing an amplification reaction from a nucleic acid preparation derived from said sample using at least one pair of primers capable of amplifying at least one representative region of said genus, and detecting the amplification product, wherein the one or more reference values are understood to refer to the concentration and/or the total amount of each bacterium belonging to one or more of the genera proposed, in the general population or in a healthy subject.

2. The *in vitro* method for determining the risk that a subject has colorectal cancer according to claim 1, wherein the method comprises:
a. determining the levels or the concentration of at least the bacteria belonging to the genus *Fusobacterium, Parvimonas, Bacteroides and Faecalibacterium,* in an intestinal or stool sample isolated from said subject; and
b. comparing the levels or the concentration of said bacteria in said intestinal or stool sample with a reference value, wherein an increase in the number of sequences of *Fusobacterium, Parvimonas, and*/*or Bacteroides,* and a decreased in the number of sequences of *Faecalibacterium,* in said sample with respect to said one or more reference values is indicative of an increase in the risk that the subject has colorectal cancer.

3. An *in vitro* method for diagnosing colorectal cancer in a subject in need thereof, comprising the following steps:
c. determining the levels or the concentration of bacteria belonging to the genus *Fusobacterium, Parvimonas, Bacteroides and*/*or Faecalibacterium,* or any combination thereof, in an intestinal or stool sample isolated from said subject; and
d. comparing the levels or the concentration of said bacteria in said intestinal or stool sample with one or more reference values, wherein an increase in the number of sequences of *Fusobacterium, Parvimonas, and*/*or Bacteroides,* and a decreased in the number of sequences of *Faecalibacterium,* in said sample with respect to said one or more reference values is indicative that the subject has colorectal cancer;
wherein the levels or the concentration of bacteria belonging to the genus *Fusobacterium, Parvimonas, Bacteroides and*/*or Faecalibacterium,* are determined by performing an amplification reaction from a nucleic acid preparation derived from said sample using at least one pair of primers capable of amplifying at least one representative region of said genus, and detecting the amplification product,
wherein the one or more reference values are understood to refer to the concentration and/or the total amount of each bacterium belonging to one or more of the genera proposed, in the general population or in a healthy subject.

4. The *in vitro* method for diagnosing colorectal cancer in a subject in need thereof according to claim 3, wherein the method comprises the following steps:
a) determining the levels or the concentration of the bacteria belonging to the genus *Fusobacterium, Parvimonas, Bacteroides and Faecalibacterium,* in an intestinal or stool sample isolated from said subject; and
b) comparing the levels or the concentration of said bacteria in said intestinal or stool sample with a reference value, wherein an increase in the number of sequences of *Fusobacterium, Parvimonas, and*/*or Bacteroides,* and a decreased in the number of sequences of *Faecalibacterium,* in said sample with respect to said one or more reference values is indicative that the subject has colorectal cancer.

5. An in vitro method for determining the risk that a subject has colorectal cancer, comprising the following steps:
c. determining the levels or the concentration of bacteria belonging to the genus *Fusobacterium, Parvimonas, Bacteroides and*/*or Faecalibacterium,* or any combination thereof, in an intestinal or stool sample isolated from said subject; and
d. identifying the subject as a subject at risk of developing colorectal cancer by a predictive model which correlates at least one or more of the levels identified in step (a) with representative levels of the same from samples obtained or isolated from subjects previously identified as suffering from colorectal cancer, said predictive model having been generated by training a computer with a plurality of the concentration levels of the bacteria belonging to the genus identified in step (a) from previously identified subjects having colorectal cancer, by machine learning on said plurality of levels so as to obtain representative score profiles associated with colorectal cancer.

6. An in vitro method for determining the risk that a subject has colorectal cancer according to claim 5, wherein the method comprises the following steps:
a) determining the levels or the concentration of bacteria belonging to at least all of the following genus: *Fusobacterium, Parvimonas, Bacteroides and Faecalibacterium,* in an intestinal or stool sample isolated from said subject; and
b) identifying the subject as a subject at risk of developing colorectal cancer by a predictive model which correlates at least one or more of the levels identified in step (a) with representative levels of the same from samples obtained or isolated from subjects previously identified as suffering from colorectal cancer, said predictive model having been generated by training a computer with a plurality of the concentration levels of the bacteria belonging to the genus identified in step (a) from previously identified subjects having colorectal cancer, by machine learning on said plurality of levels so as to obtain representative score profiles associated with colorectal cancer.

7. An in vitro method for diagnosing colorectal cancer in a subject in need thereof, comprising the following steps:
c. determining the levels or the concentration of bacteria belonging to the genus *Fusobacterium, Parvimonas, Bacteroides and*/*or Faecalibacterium,* or any combination thereof, in an intestinal or stool sample isolated from said subject; and
d. identifying the subject as a subject having colorectal cancer by a predictive model which correlates at least one or more of the levels identified in step (a) with representative levels of the same from samples obtained or isolated from subjects previously identified as suffering from colorectal cancer, said predictive model having been generated by training a computer with a plurality of the concentration levels of the bacteria belonging to the genus identified in step (a) from previously identified subjects having colorectal cancer, by machine learning on said plurality of levels so as to obtain representative score profiles associated with colorectal cancer.

8. An in vitro method for diagnosing colorectal cancer in a subject in need thereof according to claim 7, wherein the method comprises the following steps:
a) determining the levels or the concentration of bacteria belonging to at least all of the following genus: *Fusobacterium, Parvimonas, Bacteroides and Faecalibacterium,* in an intestinal or stool sample isolated from said subject; and
b) identifying the subj ect as a subj ect having colorectal cancer by a predictive model which correlates at least one or more of the levels identified in step (a) with representative levels of the same from samples obtained or isolated from subjects previously identified as suffering from colorectal cancer, said predictive model having been generated by training a computer with a plurality of the concentration levels of the bacteria belonging to the genus identified in step (a) from previously identified subjects having colorectal cancer, by machine learning on said plurality of levels so as to obtain representative score profiles associated with colorectal cancer.

9. The method according to any one of claims 1 to 4, wherein the amplification reaction is carried out by means of a real-time polymerase chain reaction.

10. The method according to any one of claims 5 or 8, wherein the levels or the concentration of bacteria belonging to the genus *Fusobacterium, Parvimonas, Bacteroides and*/*or Faecalibacterium* as identified in step (a), are determined by performing an amplification reaction from a nucleic acid preparation derived from said sample using at least one pair of primers capable of amplifying at least one representative region of said genus and detecting the amplification product.

11. The method according to any of claims 1 to 10, wherein said method further comprises storing the results of the method in a data carrier, preferably wherein said data carrier is a computer readable medium.

12. A computer-implemented method for determining the risk that a subject has colorectal cancer, wherein said method comprises at least the comparative step (step (b)) and optionally the provision of a result as a consequence of said comparison, as these steps are defined in the method according to any of claims 1 to 4.

13. A computer-implemented method for determining the risk that a subject has colorectal cancer, wherein said method comprises at least the comparative step (step (b)) and optionally the provision of a result as a consequence of said comparison, as these steps are defined in the method according to any of claims 5 to 8.

14. A kit capable of implementing the methodology described in any of claims 1 to 11.
